# EUROPEAN PATENT SPECIFICATION

(11) **EP 2 621 927 B1**
(45) Date of publication and mention of the grant of the patent: **12.02.2020**
(21) Application number: 11761589.8
(22) Date of filing: 27.09.2011
(51) Int. Cl.: C07D 487/04, C07D 519/00, H01B 1/12, H01L 51/00

(54) **SEMICONDUCTORS BASED ON DIKETOPYRROLOPYRROLES**
AUF DIKETOPYRROLOPYRROLEN BASIERENDE HALBLEITER
SEMICONDUCTEURS A BASE DE DIKETOPYRROLOPYRROLES

(30) Priority: 08.03.2011 EP 11157264; 29.09.2010 EP 10182051
(43) Date of publication of application: 07.08.2013
(73) Proprietor: BASF SE, 67056 Ludwigshafen am Rhein (DE)
(72) Inventor: WÜRTHNER, Frank, 97204 Höchberg (DE); SURARU, Sabin-Lucian, 97072 Würzburg (DE); HAYOZ, Pascal, CH-4114 Hofstetten (CH)
(74) Representative: Leybach, Holger Hugo
(86) International application number: PCT/EP2011/066763
(87) International publication number: WO 2012/041849

(56) References cited:
- WO-A1-2010/049321
- WO-A2-2009/047104

## Description

The present invention relates to 1,4-diketopyrrolo[3,4-c]pyrrole (DPP) derivatives of the below formula **(Ia)**, **(Ib)**, **(Ic),** or **(Id),** wherein the substituents are as defined herein below, to their manufacture; to their use as organic semiconductors, e.g. in semiconductor devices, especially a sensor, a diode, a photodiode, an organic field effect transistor, a transistor for flexible displays, and/or a solar cell (photovoltaic cell); to such semiconductor devices comprising diketo-pyrrolopyrrol derivatives of the formula **(Ia)**, **(Ib)**, **(Ic),** or **(Id)** as a semiconducting effective means, and to devices containing said semiconductor devices.

Prashant Sonar et al., J. Mater. Chem., 2010, 20, 3626-3636 describes novel low bandgap solution processable diketopyrrolopyrrole (DPP) based derivatives functionalized with electron withdrawing end capping groups (trifluoromethylphenyl and trifluorophenyl):

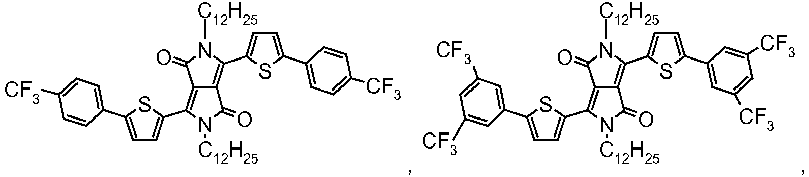

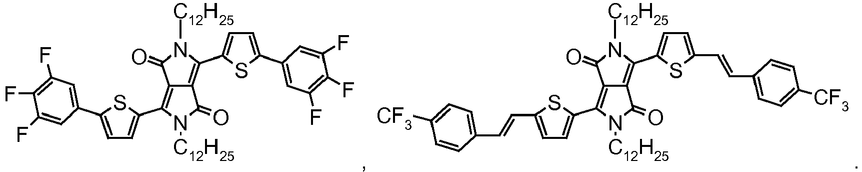

These compounds showed optical bandgaps ranging from 1.81 to 1.94 eV and intense absorption bands that cover a wide range from 300 to 700 nm, attributed to charge transfer transition between electron rich phenylene-thienylene moieties and the electron withdrawing diketopyrrolopyrrole core. All of the compounds were found to be fluorescent in solution with an emission wavelength ranging from 600 to 800 nm. Bulk heterojunction (BHJ) solar cells using poly(3-hexylthiophene) (P3HT) as the electron donor with these new acceptors were fabricated.

H. Yanagisawa et al., Japan Journal of Applied Physics 47 (2008) 4728-4731 discloses OFETs based on unsubstituted DPP or quinacridone using their solvent soluble precursors called latent pigment. The use of latent pigments enables to fabricate OFETs by spin-coating. A DPP-Pigment with a lactam-NH-group ( 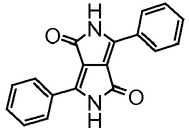 ) deposited in vacuum was compared to the Boc-protected analogue ( 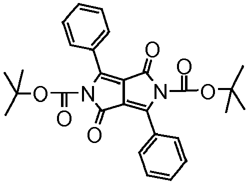 ), which was spin-coated from solution and treated afterwards at higher temperature to deprotect the carbamate. Thereby hole-mobilities of ∼10⁻⁵ and ∼10⁻⁶ cm² V⁻¹ s⁻¹ were measured respectively.

Thuc-Quyen Nguyen et al., J. Phys. Chem. C 2008, 112, 17402-17407 discloses two new oligo-thiophene derivatives bearing a diketopyrrolopyrrole core, 2,5-di-*n*-hexyl-3,6-bis(5-*n-*hexyl[2,2';5',2"]terthiophen-5-yl)pyrrolo[3,4-*c*]pyrrole-1,4-dione (DHT6DPPC6) and 2,5-di-*n-*dodecyl-3,6-bis(5"-*n*-hexyl[2,2';5',2"]terthiophen-5-yl)pyrrolo[3,4-*c*]pyrrole-1,4-dione (DHT6DPPC12), and their use in solution-processed organic field effect transistors. The field effect mobilities for annealed DHT6DPPC6 and DHT6DPPC12 films are 0.02 and 0.01 cm²/V s, respectively.

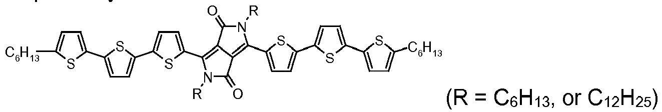

C. Winnewisser et al., Advanced Materials 20 (2008) 2217-2224 discloses a polymer semiconductor, 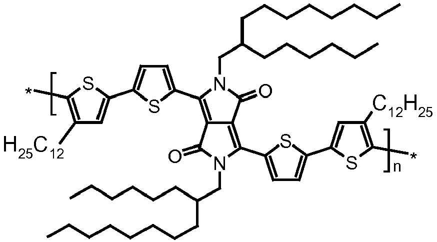 (BBTDPP1), with ambipolar charge transport properties. Ambipolar field-effect transistors based on this material exhibit hole and electron mobilities of 0.1 cm² V⁻¹ s⁻¹ and up to 0.09 cm² V⁻¹ s⁻¹, respectively.

R. A. J. Janssen et al., J. Am. Chem. Soc. 131 (2009) 16616-16617 report that 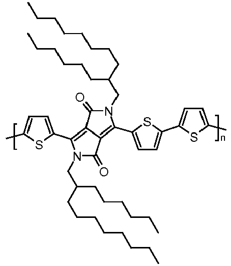 (PDPP3T) exhibits ambipolar transport in FETs with nearly balanced electron and hole mobilities in the 10⁻² cm² V⁻¹ s⁻¹ range, making it an interesting candidate for CMOS-like circuits. At a high molecular weight, PDPP3T reaches *η* = 4.7% in photovoltaic cells when combined with [70]PCBM and has a photoresponse up to 900 nm.

R. A. J. Janssen et al., Macromol. Rapid Commun. 31 (2010) 1554-1559 disclose small molecule DPP derivatives ( 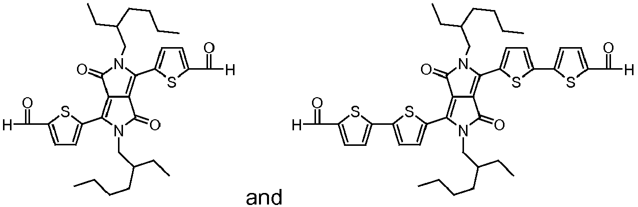 ). The compounds are tested as electron acceptors in combination with poly(3-hexylthiophene) (P3HT) as the donor material. Working photovoltaic devices are obtained that show a photoresponse in the wavelength region where the DPP molecules absorb. The best device shows a power conversion efficiency of 0.31% in simulated solar light, with a photon-to-electron conversion efficiency of ca. 10% up to 700 nm.

WO2004/090046 relates to fluorescent diketopyrrolopyrroles of the formula I 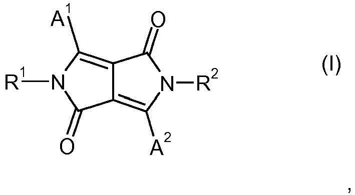 wherein
R¹ and R² may be the same or different and are selected from a C₁-C₂₅alkyl group, which can be substituted by fluorine, chlorine or bromine, an allyl group, which can be substituted one to three times with C₁-C₄alkyl, a cycloalkyl group, a cycloalkyl group, which can be condensed one or two times by phenyl which can be substituted one to three times with C₁-C₄-alkyl, halogen, nitro or CN, an alkenyl group, a cycloalkenyl group, an alkynyl group, a haloalkyl group, a haloalkenyl group, a haloalkynyl group, a ketone or aldehyde group, an ester group, a carbamoyl group, a ketone group, a silyl group, a siloxanyl group, A³ or -CR³R⁴-(CH₂)ₘ-A³ wherein R³ and R⁴ independently from each other stand for hydrogen or C₁-C₄alkyl, or phenyl which can be substituted one to three times with C₁-C₄alkyl,
A³ stands for aryl or heteroaryl, in particular phenyl or 1- or 2-naphthyl which can be substituted one to three times with C₁-C₈alkyl and/or C₁-C₈alkoxy, and m stands for 0, 1, 2, 3 or 4,
A¹ and A² are independently of each other 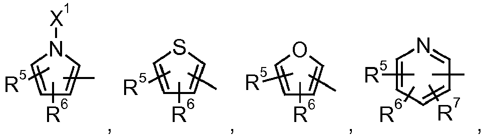 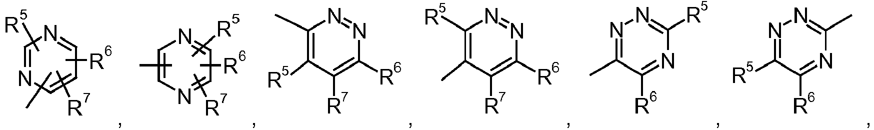 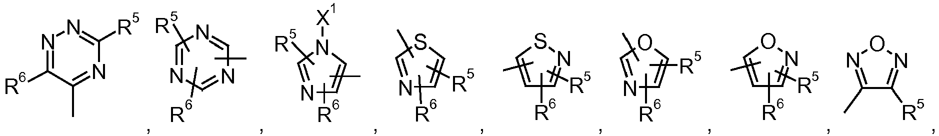 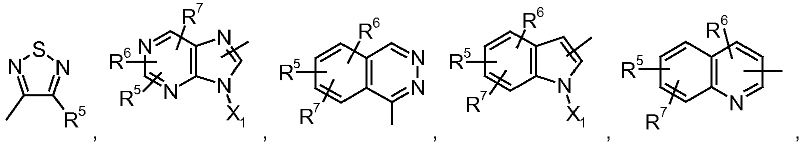 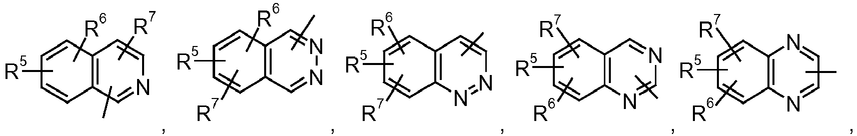 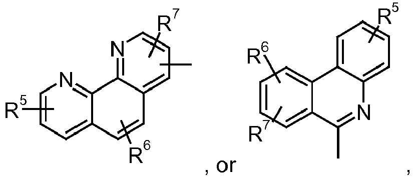 or
A¹ and A² are independently of each other a group 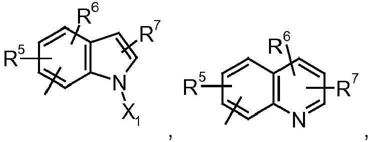 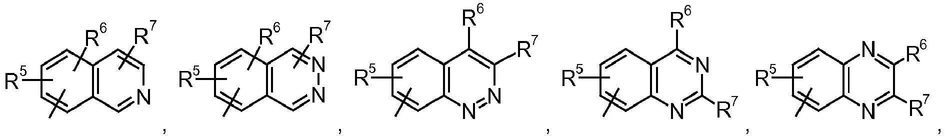 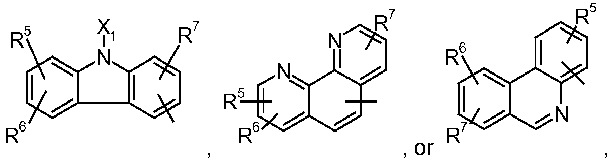 wherein
   R⁵, R⁶, and R⁷ may be the same or different and are selected from a hydrogen atom, a C₁-C₂₅alkyl group, a cycloalkyl group, an aralkyl group, an alkenyl group, a cycloalkenyl group, an alkynyl group, a hydroxyl group, a mercapto group, an alkoxy group, an alkylthio group, an aryl ether group, an aryl thioether group, an aryl group, a heterocyclic group, a halogen atom, a haloalkyl group, a haloalkenyl group, a haloalkynyl group, a cyano group, an aldehyde group, a carboxyl group, an ester group, a carbamoyl group, a nitro group, a silyl group, a siloxanyl group, a substituted or unsubstituted vinyl group, a group NR⁸R⁹, wherein R⁸ and R⁹ independently of each other stand for a hydrogen atom, an alkyl group, a cycloalkyl group, an aryl group, a heteroaryl group, a heterocyclic group, an aralkyl group, or R⁸ and R⁹ together with the nitrogen atom to which they are bonded form a five or six membered heterocyclic ring, which can be condensed by one or two optionally substituted phenyl groups, or at least two adjacent substituents R⁵ to R⁷ form an aromatic or aliphatic fused ring system, and
   X¹ is a hydrogen atom, a C₁-C₂₅alkyl group, a cycloalkyl group, an aralkyl group, an aryl group, or a heterocyclic group, wherein at least one of the groups R⁵, R⁶, and R⁷ is different from a hydrogen atom, if A¹ and A² are a single five- or six-membered heterocyclic ring, containing one heteroatom selected from the group of nitrogen, oxygen and sulfur.

JP2006117591A relates to compounds of formula 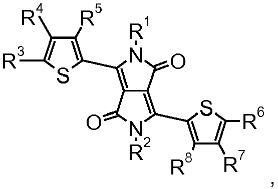 wherein R¹, R² = (un)substituted alkyl group, aryl group or heterocyclic group; R³-R⁸ = H, halogen, cyano group, (un)substituted alkyl group, alkoxy group, aryl group, aryloxy group, alkyl thio group, aryl thio group, alkyl carbonyl group, aryl carbonyl group or amino group; X¹, X² = oxygen, sulfur or - NR⁹;and R⁹ = H, (un)substituted alkyl, aryl or heterocyclic group, or R¹-R⁹ are combined to form a ring, and their use in organic electroluminescent elements. Among others the following compounds are explicitly mentioned: 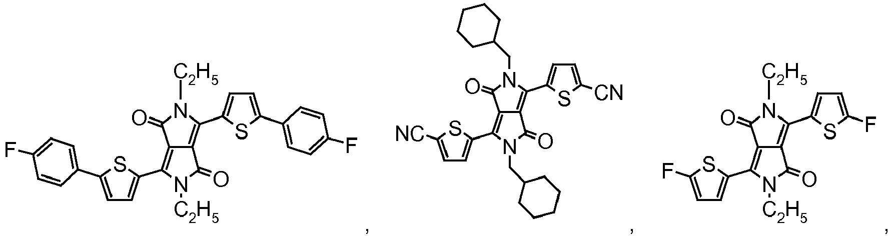 and

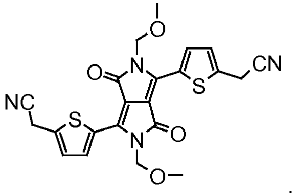

JP2007266285 relates to OFETs which use, as semiconductors, compounds of 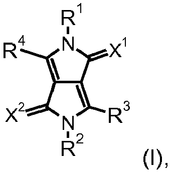 where X¹, X² = O, S or Se; and R¹-R⁴ = H, or aliph. hydrocarbon or arom. groups which may be substituted. The following compounds were explicitly disclosed: 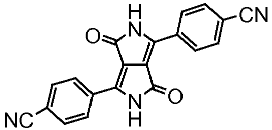 and

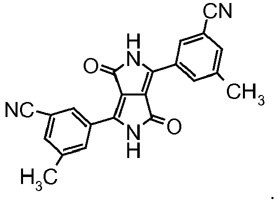

JP2008078247 relates to an organic transistor having an di-Ph pyrrolopyrroledione organic semiconductor compound, formed by vacuum vapor deposition. The following compounds were explicitly disclosed:

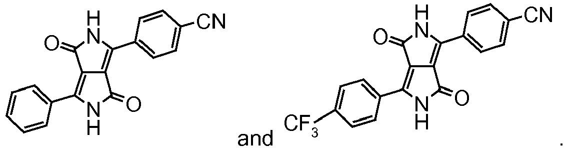

EP2034537A2 relates to a thin film transistor device comprising a semiconductor layer, the semiconductor layer comprising a compound comprising a chemical structure represented by: 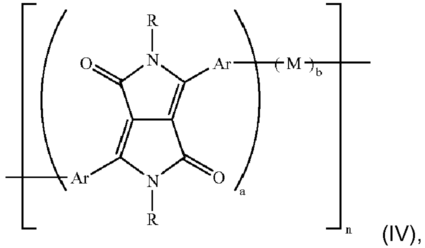 wherein each R is independently selected from hydrogen, an optionally substituted hydrocarbon, and a hetero-containing group; each Ar is independently selected from optionally substituted aryl and heteroaryl groups; each M is an optional, conjugated moiety; a represents a number that is at least 1; b represents a number from 0 to 20; and n represents a number that is at least 1.

WO2009/047104 relates to a compound of the formula I 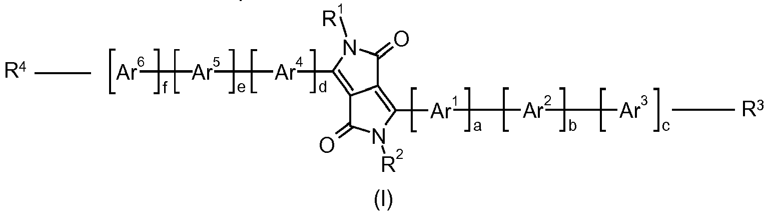
wherein R¹ and R² are independently of each other an aliphatic, cycloaliphatic, cycloaliphatic-aliphatic, aromatic, aromatic-aliphatic, heteroaromatic or heteroaromatic-aliphatic group having up to 49 carbon atoms,
a and d independently of each other are 0, 1, 2 or 3,
Ar¹ and Ar⁴ are independently of each other a bivalent group of the formula II or IV 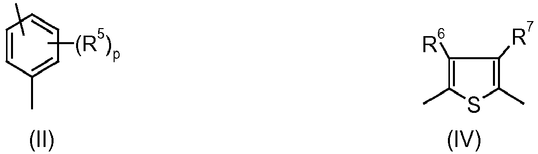
   wherein R⁶ and R⁷ are as defined below,
   p represents 0, 1, or 2,
   R⁵ is an aliphatic hydrocarbon group having up to 25 carbon atoms, or two vicinal groups R⁵ together represent alkylene or alkenylene having up to 7 carbon atoms, it being possible that two groups R⁵ present in the group of formula II differ from each other,
   b, c, e, and f independently of each other represent 1, 2 or 3,
   Ar², Ar³, Ar⁵, and Ar⁶ are independently of each other a bivalent group of one of the formulae 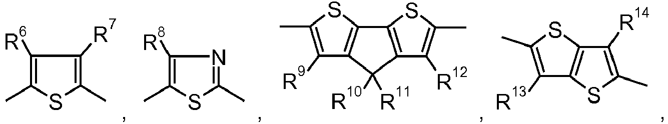 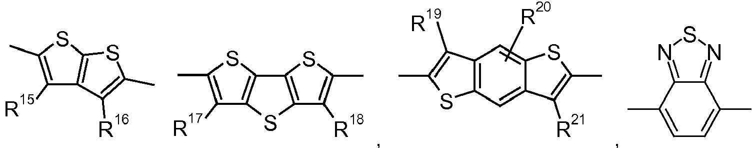
      wherein R⁶, R⁷, R⁸, R⁹, R¹², R¹³, R¹⁵, R¹⁶, R¹⁷, R¹⁸, R¹⁹, R²⁰ and R²¹ are independently of each other hydrogen, C₁-C₂₅alkyl, C₁-C₁₈alkoxy, C₆-C₂₄aryl, C₇-C₂₅aralkyl, or heteroaryl, or R⁶ and R⁷ together represent alkylene or alkenylene which may be both bonded via oxygen and/or sulfur to the thienyl residue and which may both have up to 25 carbon atoms,
      R¹⁰ and R¹¹ are independently of each other hydrogen, C₁-C₁₈alkyl, C₆-C₂₄aryl, heteroaryl, or R¹⁰ and R¹¹ together represent oxo or form a five or six membered ring, which is unsubstituted or substituted by
         a) an aliphatic hydrocarbon group having up to 18 carbon atoms,
         b) C₁-C₁₈alkoxy or C₂-C₁₈alkylenedioxy in both of which carbon atoms which are not adjacent to oxygen may be replaced by oxygen, or
         c) C₆-C₂₄aryl, C₇-C₂₅aralkyl, heteroaryl, C₃-C₁₂cycloalkyl or C₄-C₁₂cycloalkyl-alkyl, and
      R³ and R⁴ are independently of each other a group of one of the formulae 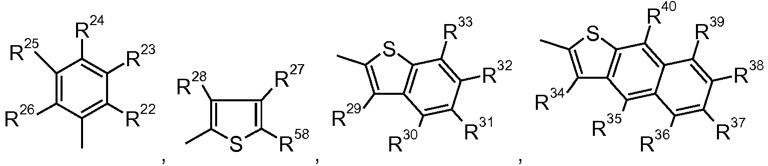 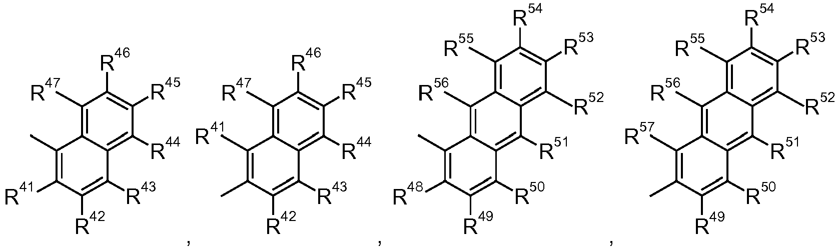 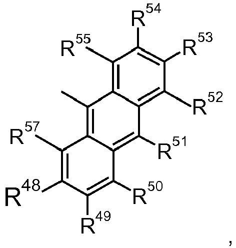 wherein R²² to R²⁶ and R²⁹ to R⁵⁸ represent independently of each other hydrogen, an aliphatic hydrocarbon group having up to 25 carbon atoms, alkoxy or alkenyloxy having up to 18 carbon atoms, halogen, a cycloaliphatic, cycloaliphatic-aliphatic, aromatic, aromatic-aliphatic, heteroaromatic or heteroaromatic-aliphatic group having up to 25 carbon atoms, or a group of the formula 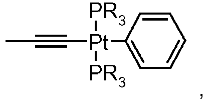 wherein R represents an aliphatic hydrocarbon group having up to 12 carbon atoms, or two groups R²² to R²⁶ and R²⁹ to R⁵⁷ which are in the neighborhood of each other, together represent alkylene or alkenylene having up to 8 carbon atoms, thereby forming a ring, and
      R²⁷ and R²⁸ are independently of each other hydrogen, C₁-C₂₅alkyl, C₁-C₁₈alkoxy, C₆-C₂₄aryl, C₇-C₂₅aralkyl, heteroaryl, or a group of the formula (III) shown above, wherein R represents an aliphatic hydrocarbon group having up to 12 carbon atoms, or R²⁷ and R²⁸ together or R²⁷ and R⁵⁸ together represent alkylene or alkenylene which may be both bonded via oxygen and/or sulfur to the thienyl residue and which may both have up to 25 carbon atoms.

PCT/EP2010/053655 relates to compounds of the formula 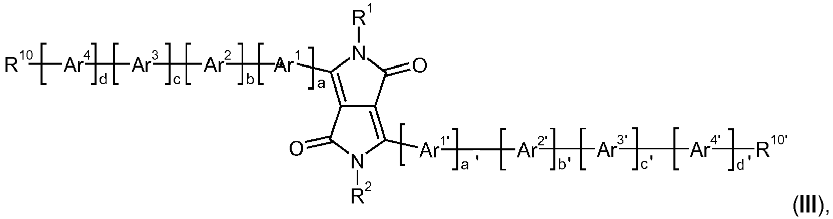
wherein a, a', b, b', c, c', d, d', R¹, R², Ar¹, Ar^{1'}, Ar², Ar^{2'}, Ar³, Ar^{3'}, Ar⁴ and Ar^{4'} are as defined in claim 1,
R¹⁰ and R^{10'} are independently of each other hydrogen, halogen, C₁-C₂₅alkyl, C₁-C₂₅alkoxy, or a group of one of the formulae **IVa** to **IVi,** 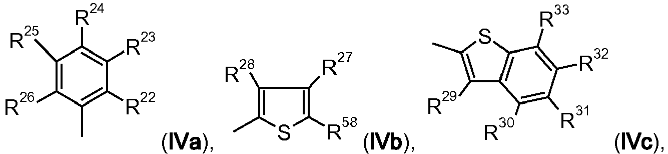 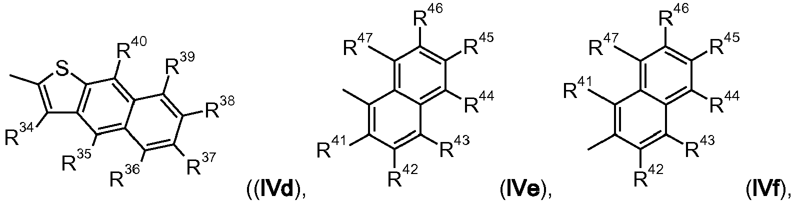 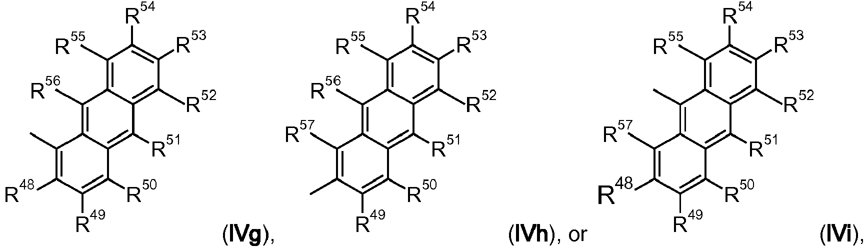
   wherein R²² to R²⁶ and R²⁹ to R⁵⁸ represent independently of each other H, halogen, C₁-C₂₅alkyl, C₁-C₂₅alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, a C₄-C₁₈cycloalkyl group, a C₄-C₁₈cycloalkyl group, which is substituted by G, C₂-C₁₈alkenyl, C₂-C₁₈alkynyl, C₁-C₁₈alkoxy, C₁-C₁₈alkoxy which is substituted by E and/or interrupted by D, C₇-C₂₅aralkyl, or C₇-C₂₅aralkyl, which is substituted by G,
   R²⁷ and R²⁸ are independently of each other hydrogen, C₁-C₂₅alkyl, or C₇-C₂₅aralkyl, or R²⁷ and R²⁸ together represent alkylene or alkenylene which may be both bonded via oxygen and/or sulfur to the thienyl residue and which may both have up to 25 carbon atoms,
   D is -CO-, -COO-, -S-, -O-, or -NR¹¹²-,
   E is C₁-C₈thioalkoxy, C₁-C₈alkoxy, CN, -NR¹¹²R¹¹³, -CONR¹¹²R¹¹³, or halogen,
   G is E, or C₁-C₁₈alkyl, and
   R¹¹² and R¹¹³ are independently of each other H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; with the proviso that the following compound 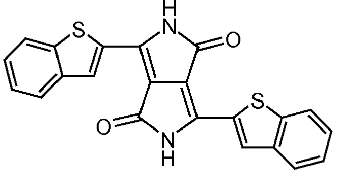 is excluded.

US2010/0032657 describes an org. transistor comprising a source electrode, a drain electrode, a gate electrode, and an org. semiconductor layer, characterized in that the organic semiconductor layer comprises a compound represented by the following general formula 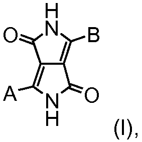 wherein A and B each independently represent a substituted alkyl group, an unsubstituted alkyl group, a substituted heterocyclic group, an unsubstituted heterocyclic group, a substituted aryl group, or an unsubstituted aryl group; or general formula 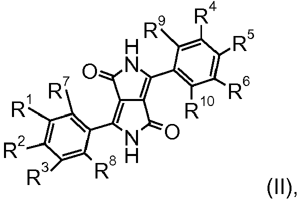 wherein R¹ to R¹⁰ are each independently a hydrogen atom, a halogen atom, an alkyl group which has 4 or less carbon atoms, an alkyl group which has 4 or less carbon atoms that is substituted with a halogen atom, an alkoxy group which has 4 or less carbon atoms, an alkoxy group which has 4 or less carbon atoms that is substituted with a halogen atom, an amino group which has 4 or less carbon atoms, an amino group which has 4 or less carbon atoms that is substituted with a halogen atom, a nitro group, or a cyano group, and the compound contains at least one halogen atom. Among others the following compounds are explicitly mentioned: 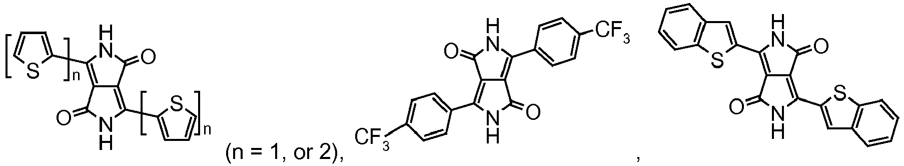 and

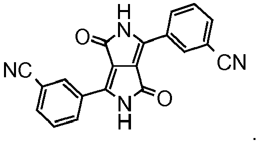

WO2008/013427A1 relates to organic thin film transistors comprising an organic semiconductor layer including an organic semiconductor substance having a functional group that can form a hydrogen bond. Examples of the organic semiconductor substance are represented by compounds of formula 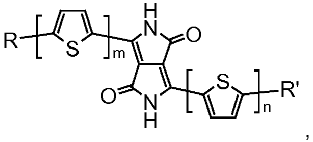 wherein n and m are each independently 0 or a positive integer, and R and R' are each independently any one selected from the group consisting of an alkyl group, an alkoxy group, an acetyl group, an imine group, an ether group, an ester group, a nitrile group, a thioalkoxy group, an amino group, a thioester group, a vinyl group, an aryl group, and a hetero group.

It is the object of the present invention to provide compounds, which show high efficiency of energy conversion, excellent field-effect mobility, good on/off current ratios and/or excellent stability, when used in organic field effect transistors, organic photovoltaics (solar cells) and photodiodes.

Said object has been solved by compounds of the formula 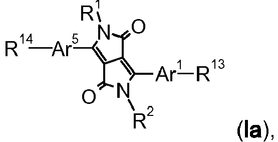 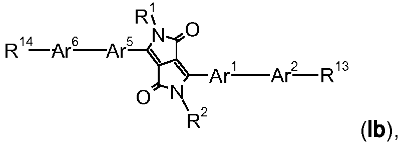 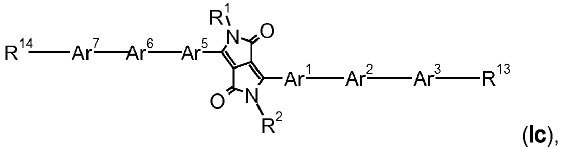 or 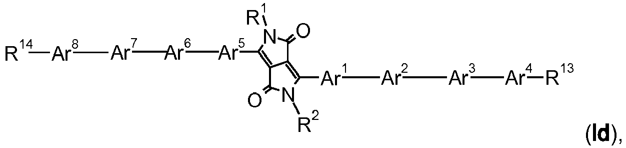 wherein
R¹ and R² are selected from a C₁-C₃₈alkyl group, a C₁-C₃₈alkyl group which is substituted by one or more halogen atoms, especially a F containing C₂-C₃₆alkyl group, very especially CH₂CₙF₂ₙ₊₁ (n = 1 to 10); a C₇-C₂₅arylalkyl group, which can be substituted one to five times with C₁-C₈alkyl, C₁-C₈alkoxy and/or F, especially 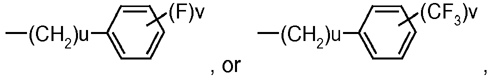 u is 1, or 2, v is 1 to 5; and pentafluorophenyl;
Ar¹ and Ar⁵ are independently of each other 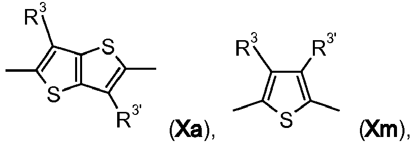 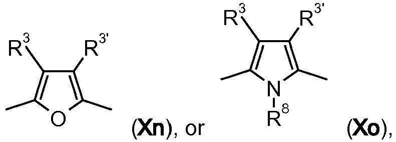 wherein R³ and R^{3'} are independently of each other hydrogen, halogen, CF₃, CN, C₁-C₂₅alkyl or C₁-C₂₅alkoxy, and R⁸ is hydrogen or C₁-C₂₅alkyl; Ar², Ar³, Ar⁴, Ar⁶, Ar⁷ and Ar⁸ are independently of each other 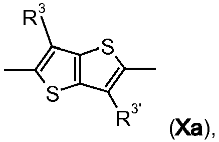 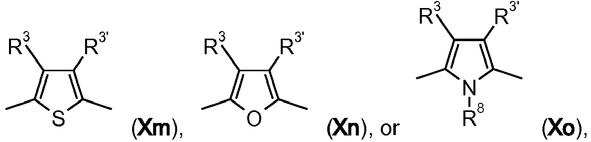 wherein R³ and R^{3'} are independently of each other hydrogen, halogen, CF₃, CN, C₁-C₂₅alkyl or C₁-C₂₅alkoxy, and R⁸ is hydrogen or C₁-C₂₅alkyl; and
R¹³ and R¹⁴ are CN, or 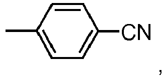 especially CN.

Advantageously, the compounds of the present invention, or an organic semiconductor material, layer or component, comprising the compounds of the present invention can be used in organic photovoltaics (solar cells) and photodiodes, or in an organic field effect transistor (OFET).

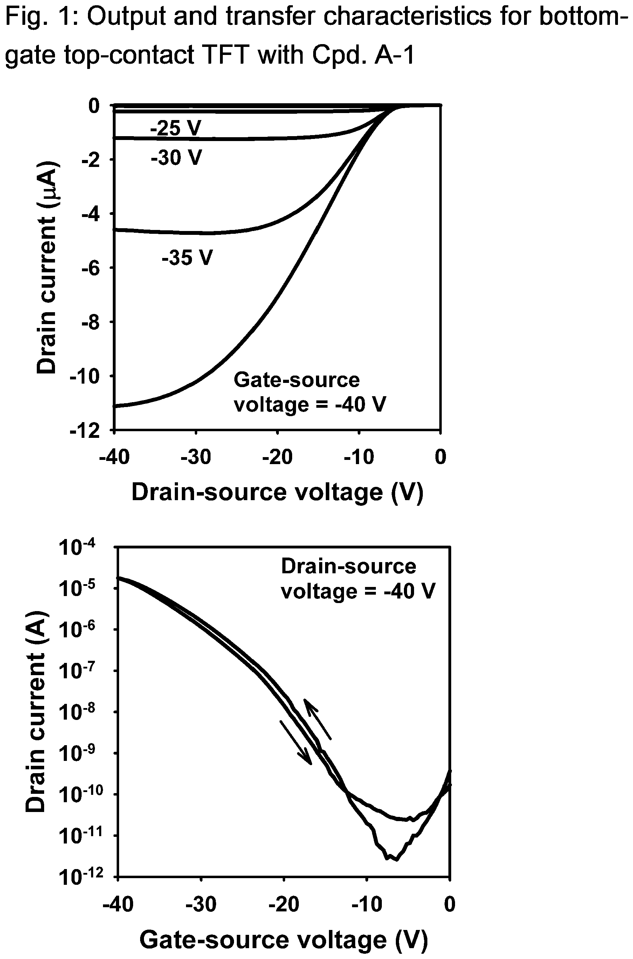

Fig. 1 shows output and transfer characteristics for bottom-gate top-contact TFT with 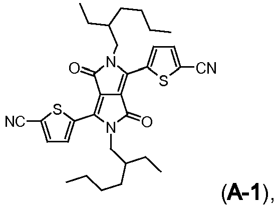 the synthesis of which is described in Example 1. The novel type of efficient p-channel material, compound **A-1,** exhibits high hole-mobilities and on/off-ratios in vacuum deposited TFTs.

R¹ and R² may be different, but are preferably the same; and are preferably selected from a C₁-C₁₀₀alkyl group, a C₁-C₁₀₀oalkyl group which is substituted by one or more halogen atoms, -CN, or C₆-C₁₈aryl groups and/or interrupted by -O-, -COO-, -OCO-, or -S-; a C₇-C₂₅arylalkyl group, which can be substituted one to five times with C₁-C₈alkyl, C₁-C₈alkoxy, -CF₃ and/or F; and pentafluorophenyl.

More preferably R¹ and R² are selected from a C₁-C₃₈alkyl group, a C₁-C₃₈alkyl group which is substituted by one or more halogen atoms, especially a F containing C₂-C₃₆alkyl group, very especially CH₂CₙF₂ₙ₊₁ (n = 1 to 10); a C₇-C₂₅arylalkyl group, which can be substituted one to five times with C₁-C₈alkyl, C₁-C₈alkoxy, -CF₃ and/or F, especially 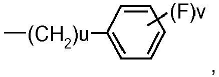 or 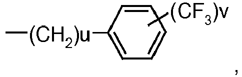 u is 1, or 2, v is 1 to 5; and pentafluorophenyl.

In a preferred embodiment R¹ and R² are a C₁-C₃₈alkyl group, especially a C₂-C₁₂alkylgroup, such as, for example, methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl 2-ethylhexyl, n-nonyl, decyl, undecyl, and n-dodecyl.

Advantageously, the groups R¹ and R² can be represented by formula 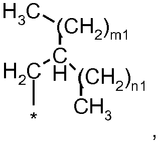 wherein m1 = n1 + 2 and m1 + n1 ≤ 24. Chiral side chains, such as R¹ and R², can either be enantiomerically pure, homochiral, or racemic, which can influence the morphology of the polymers.

In another preferred embodiment R¹ and R² are a F containing C₂-C₃₆alkyl group, especially CH₂CₙF₂ₙ₊₁ (n = 1 to 10), such as, for example, CH₂CF₃, CH₂C₂F₅, CH₂C₃F₇, CH₂C₄F₉, CH₂C₅F₁₁, CH₂C₆F₁₃ and CH₂C₁₀F₂₁.

In another preferred embodiment R¹ and R² are a C₇-C₂₅arylalkyl group, which can be substituted one to five times with C₁-C₈alkyl, C₁-C₈alkoxy and/or F. In said embodiment groups of formula 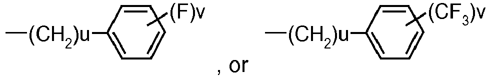 are more preferred, wherein u is 1, or 2, and v is 1 to 5. Examples are

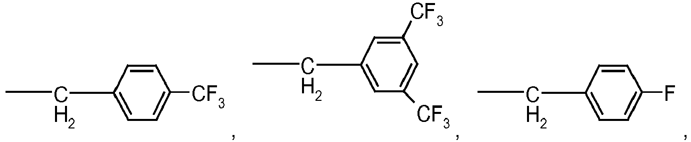

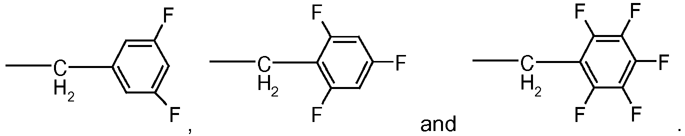

Preferably, R³ and R^{3'} are independently of each other hydrogen, halogen, CF₃, cyano, C₁-C₂₅alkyl or C₁-C₂₅alkoxy; more preferably CF₃, cyano or C₁-C₂₅alkyl; most preferred hydrogen, or C₁-C₂₅alkyl.;

Ar¹ and Ar⁵ are independently of each other a group of formula (Xa), (Xm), (Xn), or **(Xo),** preferably a group of formula **(Xa), (Xm),** or **(Xn),** especially **(Xm).**

Ar¹ and Ar⁵ are independently of each other 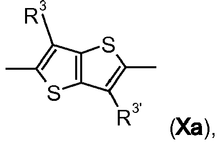 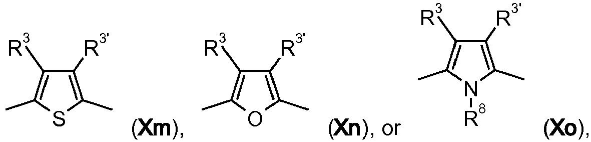 wherein R³ and R^{3'} are independently of each other hydrogen, halogen, CF₃, CN, C₁-C₂₅alkyl or C₁-C₂₅alkoxy, and R⁸ is hydrogen or C₁-C₂₅alkyl, especially C₃-C₂₅alkyl, most preferably Ar¹ and Ar⁵ are independently of each other

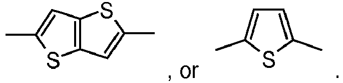

Preferably, Ar², Ar³, Ar⁴, Ar⁶, Ar⁷ and Ar⁸ are independently of each other a group of formula **(Xa), (Xm), (Xn),** or **(Xo),** preferably a group of formula **(Xa), (Xm),** or **(Xn),** especially **(Xm).**

Ar², Ar³, Ar⁴, Ar⁶, Ar⁷ and Ar⁸ are independently of each other 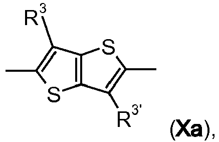 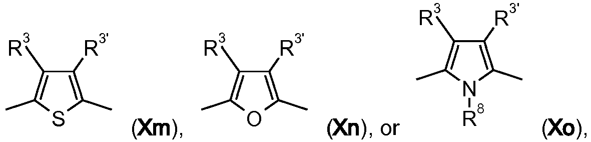 wherein R³ and R^{3'} are independently of each other hydrogen, halogen, CF₃, CN, C₁-C₂₅alkyl or C₁-C₂₅alkoxy, and R⁸ is hydrogen or C₁-C₂₅alkyl, most preferably Ar², Ar³, Ar⁴, Ar⁶, Ar⁷ and Ar⁸ are independently of each other

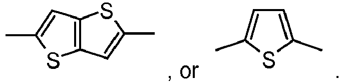

In a preferred embodiment the present invention is directed to compounds of formula 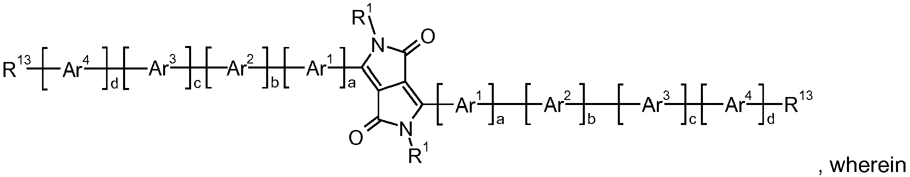 R¹ is a C₁-C₃₈alkyl group, especially a C₂-C₁₂alkyl group, a F containing C₂-C₃₆alkyl group, especially CH₂CₙF₂ₙ₊₁ (n = 1 to 10), o is an integer of 1 to 5, and p is an integer of 1 to 5, or a C₇-C₂₅arylalkyl group, which can be substituted one to five times with C₁-C₈alkyl, C₁-C₈alkoxy and/or F, especially 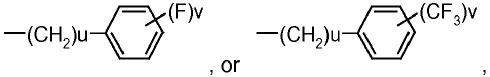 u is 1, or 2, and v is 1 to 5; 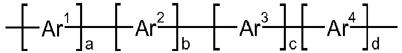 is a group of formula, or 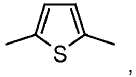 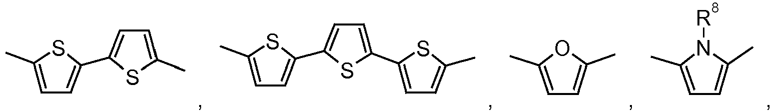 or 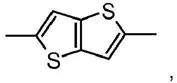 and R¹³ is CN, or

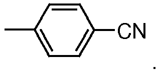

In a particularly preferred embodiment the present invention is directed to compounds of formula 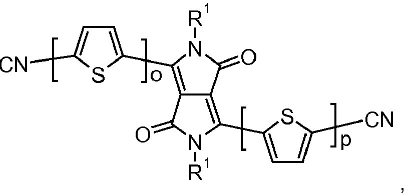 wherein R¹ is a C₁-C₃₈alkyl group, especially a C₂-C₁₂alkyl group, a F containing C₂-C₃₆alkyl group, especially CH₂CₙF₂ₙ₊₁ (n = 1 to 10), o is an integer of 1 to 5, and p is an integer of 1 to 5, or a C₇-C₂₅arylalkyl group, which can be substituted one to five times with C₁-C₈alkyl, C₁-C₈alkoxy and/or F, especially 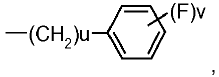 or 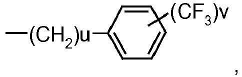 u is 1, or 2, and v is 1 to 5.

In another particularly preferred embodiment the present invention is directed to compounds of the formula 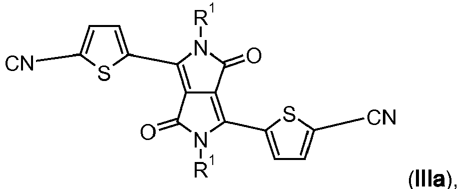 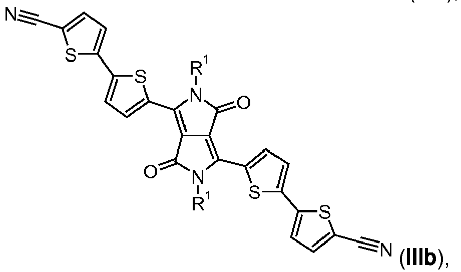 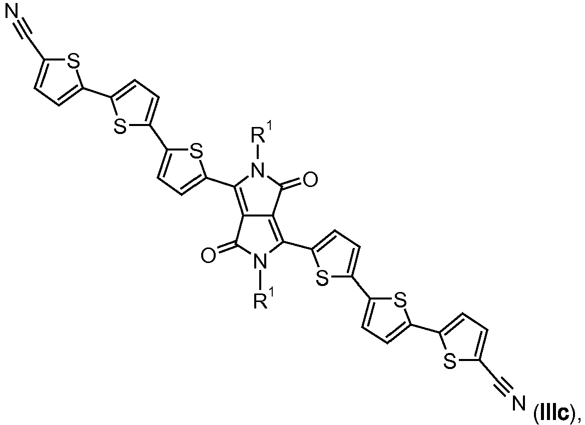 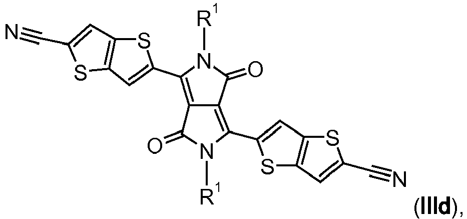 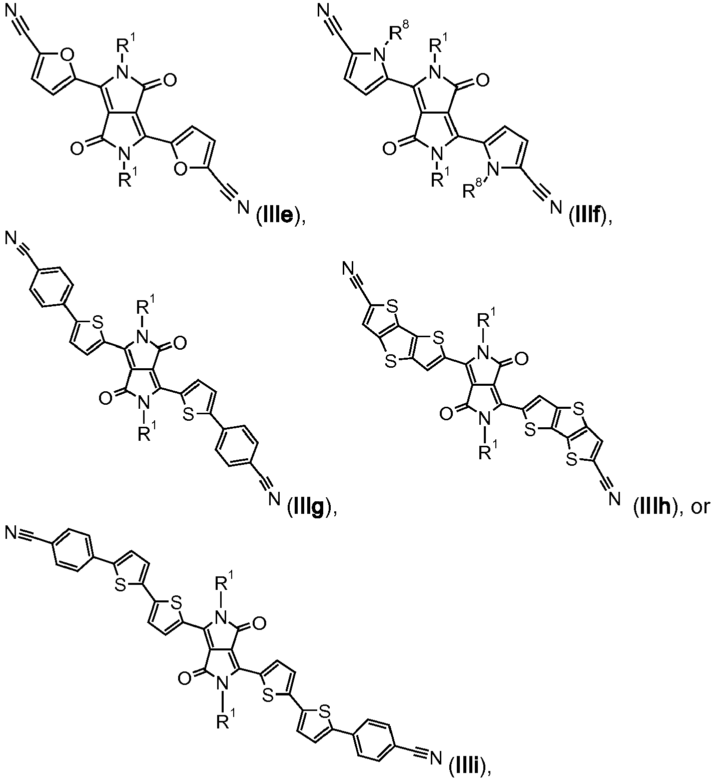 wherein R¹ is a C₁-C₃₈alkyl group, especially C₂-C₁₂alkyl group, such as, for example, methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl 2-ethylhexyl, n-nonyl, decyl, undecyl, and n-dodecyl, a F containing C₂-C₃₆alkyl group, especially CH₂CₙF₂ₙ₊₁ (n = 1 to 10), or 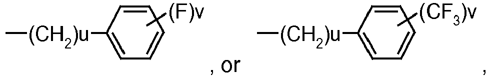 u is 1, or 2, v is 1 to 5, and R⁸ is hydrogen or C₁-C₂₅alkyl.

Compounds of formula **IIIf** are less preferred than compounds of of formula **IIIa** to **IIIe** and **IIIg** to **IIIi,** wherein compounds of formula **IIIa** to **IIIe** are particularly preferred and compounds of formula **IIIa**, **IIId** and **IIIe** are most preferred.

At present most preferred are compounds **A-1** to **A-11** and **A-13** to **A-17** and **A-19,** which are depicted in claim 10. In further embodiments of the present invention compound 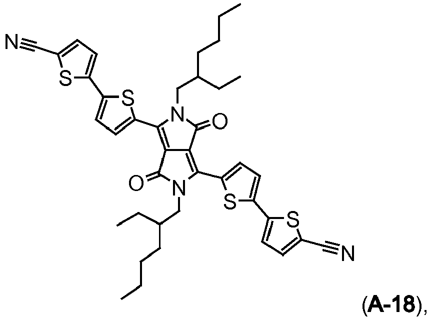 or compound 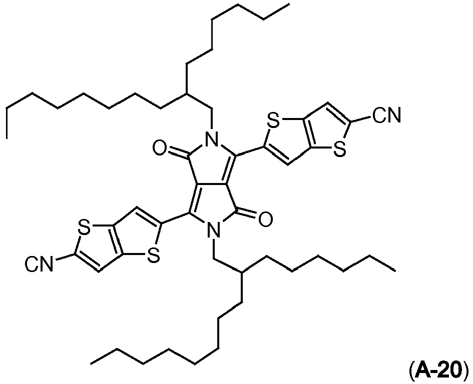 are preferred.

Compounds of formula 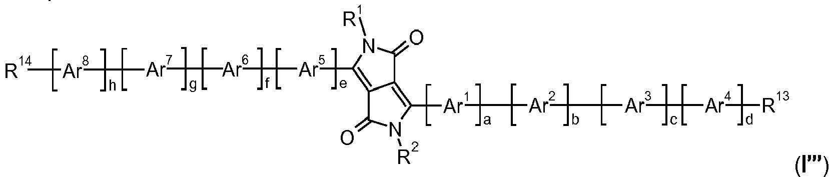 can be prepared by a process, which comprises reacting a compound of formula 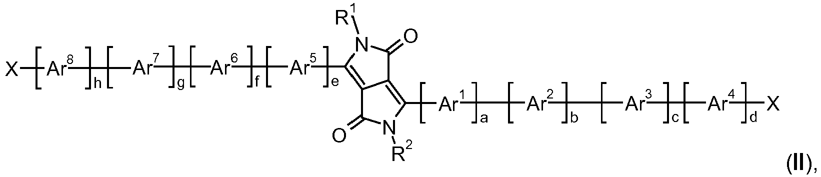 with copper(I)cyanide. Compounds of formula 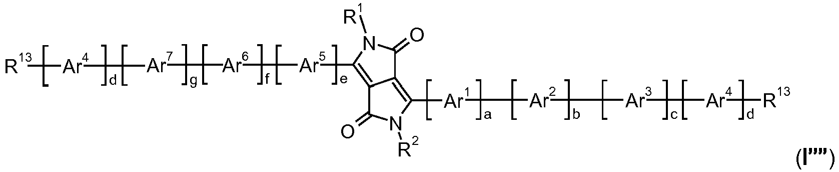 can be prepared by a process, which process comprises reacting a compound of formula 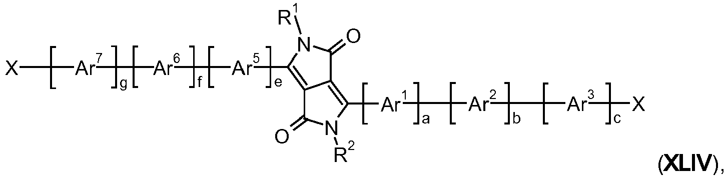 with an equimolar amount of a diboronic acid or diboronate corresponding to formula X¹¹-A²-R¹³ in a solvent and in the presence of a catalyst, or with an equimolar amount of an organo tin compound corresponding to formula X^{11'}-A²-R¹³.

The reaction of a compound of formula II with copper(I)cyanide is carried out in a suitable solvent, like dimethylforamide (DMF) and is carried out at a temperature from about room temperature to about 180°C, preferably from about 100 °C to about 170 °C, e.g. at 130 °C.

Compounds of formula 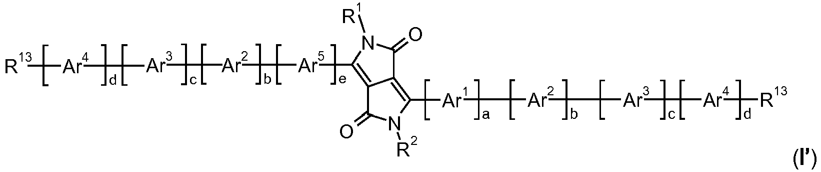 can be prepared by a process, which comprises reacting a compound of formula 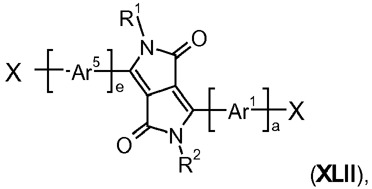 with an equimolar amount of a diboronic acid or diboronate corresponding to formula X¹¹-A-R¹³ in a solvent and in the presence of a catalyst; or
with an equimolar amount of an organo tin compound corresponding to formula X^{11'}-A-R¹³.

Compounds of formula 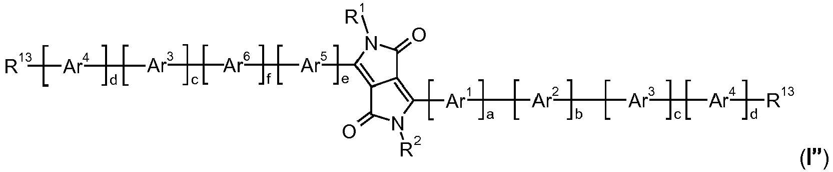 can be prepared by a process, which comprises reacting a compound of formula 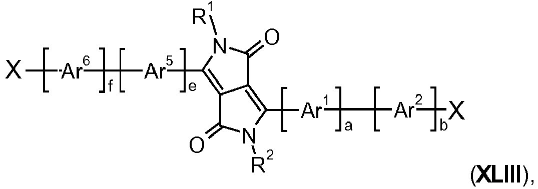 with an equimolar amount of a diboronic acid or diboronate corresponding to formula X¹¹-A¹-R¹³ in a solvent and in the presence of a catalyst; or
with an equimolar amount of an organo tin compound corresponding to formula X^{11'}-A¹-R¹³.

X¹¹ is independently in each occurrence -B(OH)₂, -B(OY¹)₂, 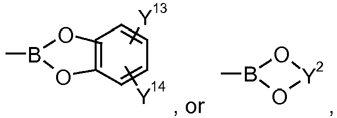 wherein Y¹ is independently in each occurrence a C₁-C₁₈alkylgroup and Y² is independently in each occurrence a C₂-C₁₀alkylene group, such as -CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, -C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup, in a solvent and in the presence of a catalyst.

The Suzuki reaction is typically conducted at about 0 °C to 180 °C in an aromatic hydrocarbon solvent such as toluene, xylene. Other solvents such as dimethylformamide, dioxane, dimethoxy-ethan and tetrahydrofuran can also be used alone, or in mixtures with an aromatic hydrocarbon. An aqueous base, preferably sodium carbonate or bicarbonate, potassium phosphate, potassium carbonate or bicarbonate is used as activation agent for the boronic acid, boronate and as the HBr scavenger. A condensation reaction may take 0.2 to 100 hours. Organic bases, such as, for example, tetraalkylammonium hydroxide, and phase transfer catalysts, such as, for example TBAB, can promote the activity of the boron (see, for example, Leadbeater & Marco; Angew. Chem. Int. Ed. Eng. 42 (2003) 1407 and references cited therein). Other variations of reaction conditions are given by T. I. Wallow and B. M. Novak in J. Org. Chem. 59 (1994) 5034-5037; and M. Remmers, M. Schulze, and G. Wegner in Macromol. Rapid Commun. 17 (1996) 239-252.

As illustrated above the compounds of the present invention can also be sythesized by the Stille coupling (see, for example, Babudri et al, J. Mater. Chem., 2004, 14, 11-34; J. K. Stille, Angew. Chemie Int. Ed. Engl. 1986, 25, 508). In order to carry out the process, the tin compounds and the halogen compounds are preferably introduced into one or more inert organic solvents and stirred at a temperature of from 0 to 200°C, preferably from 30 to 170°C for a period of from 1 hour to 200 hours, preferably from 5 hours to 150 hours.

X^{11'} is independently in each occurrence -SnR²⁰⁷R²⁰⁸R²⁰⁹, wherein R²⁰⁷, R²⁰⁸ and R²⁰⁹ are identical or different and are H or C₁-C₆alkyl, or two of the groups R²⁰⁷, R²⁰⁸ and R²⁰⁹ form a ring and these groups are optionally branched.

In formula **I'''** and **I'''** R¹³ and R¹⁴ are CN. In formula **I'** and I" R¹³ and R¹⁴ are as defined above and are preferably CN.

A is a group of formula

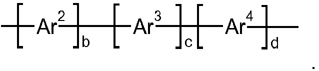

A¹ is a group of formula

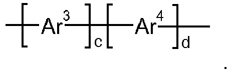

A² is a group of formula

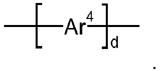

X is Cl, Br, or I; a is 1, and e is 1, b, c, d, f, g and h independently of each other represent 0, or 1, R¹, R², Ar¹, Ar², Ar³, Ar⁴, Ar⁵, Ar⁶, Ar⁷ and Ar⁸ are as defined above.

In the above described Suzuki and stille couplings the DPP compounds may alternatively comprise the diboronic acid, or diboronate, or organo tin functionalities, X¹¹ and X^{11'}, and the groups A, A¹ and A² may comprise the halogen functionality, X.

As illustrated above the compounds of the formula I can be manufactured by known methods.

A possible route of manufacture starts from a compound of the formula 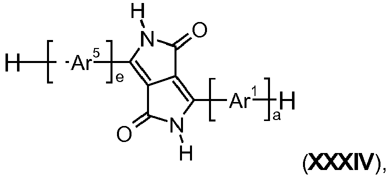 wherein a and e represent 1 and Ar¹ and Ar⁵ have the meanings given above, or from a compound of the formula 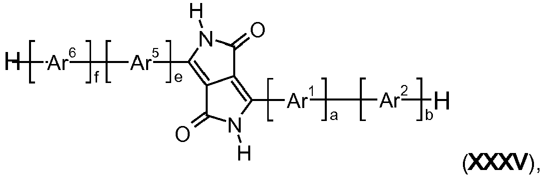 wherein a and e represent 1, b and f represent 1, and Ar¹, Ar⁵, Ar² and Ar⁶ have the meanings given above.

Said starting compounds of the formulae **XXXIV** ( 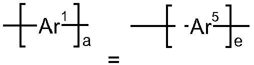 ) and **XXXV** ( 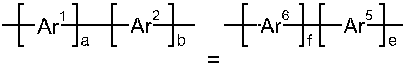 ) can be obtained as described in US4,579,949 by reacting (in the presence of a strong base) one mole of a disuccinate, like dimethyl succinate, with 1 mole of a nitrile of the formulae H-Ar¹-CN **(XXXVI)**, or H-Ar⁵⁻CN **(XXXVII)**, or
1 mole of a nitrile of the formulae H-Ar²-Ar¹-CN **(XXXVIII)**, or H-Ar⁶-Ar⁵-CN **(XXXIX).**

Alternatively, said starting compounds of the formulae **XXXIV** and **XXXV** can be obtained as described in US4,659,775 by reacting a nitrile with a suitable ester, like a pyrrolinon-3-carboxylic ester derivative.

The thus obtained compound of the formula **XXXIV** or the thus obtained compound of the formula **XXXV** is then N-alkylated for introduction of the groups R¹ and R², e.g. by reaction with a bromide of the formula R¹-Br in the presence of a suitable base, like potassium carbonate, in a suitable solvent, like N-methyl-pyrrolidone. The reaction is carried out at a temperature from about room temperature to about 180°C, preferably from about 100 °C to about 170 °C, e.g. at 140 °C.

The thus obtained compound of the formula XL 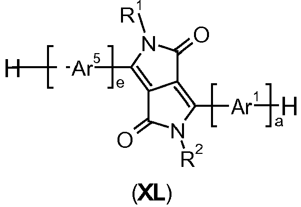 wherein a and e represent 1, and R¹, R², Ar¹ and Ar⁵ have the meanings given above, or the thus obtained compound of the formula XLI 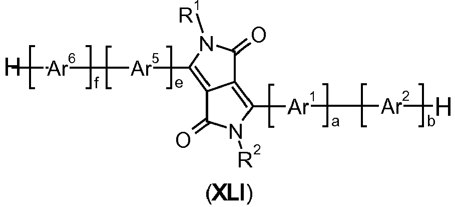 wherein a and e represent 1, b and f represent 1, and R¹, R², Ar² and Ar⁶ have the meanings given above, is then reacted with a suitable brominating agent, like N-bromo-succinimide, to yield a compound of the formulae 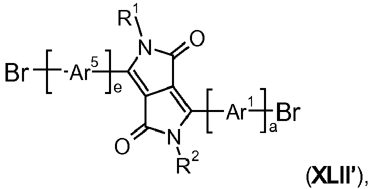 and 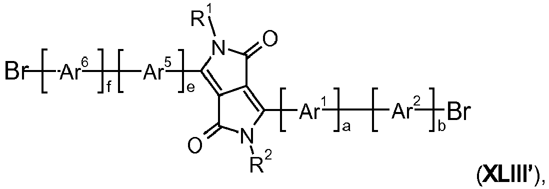 respectively.

The bromination is carried out in a suitable solvent, like chloroform, using two equivalents of N-bromo-succinimide at a temperature between -30°C and +50 °C, preferably between -10°C and room temperature, e.g. at 0°C.

The compounds of the formulae **XLII'** or **XLIII'** can then be "side-chain-elongated", by step-wise adding further groups Ar²-H, Ar⁶-H, Ar³-R¹³, and Ar⁷-R¹⁴. The step-wise addition of these groups can be effected e.g. by reacting a compound of the formulae **XLII** or **XLIII** with a suitable tin compound of the formula Ar -SnR²⁰⁷R²⁰⁸R²⁰⁹, wherein Ar represents Ar²-H, Ar⁶-H, Ar³-R¹³, or Ar⁷-R₁₄.

The reaction is carried out in the presence of a suitable palladium catalyst, like Pd(P[C₆H₅]₃)₄, in a suitable solvent, e.g. an aromatic hydrocarbon solvent, like toluene, at a temperature between about 50 °C and 180°C, e.g. under reflux, and under inert conditions including, inter alia, the use of dry solvents. After cooling down, the reaction mixture may be e.g. filtrated, e.g. on a double layer silica gel/Hyflo®, concentrated and the desired compound precipitated, e.g. by addition of methanol.

The "side-chain-elongation" of the compounds of the formulae **XLII** or **XLIII** with an additional thienyl residue can also be effected by Suzuki condensation, e.g. by reaction with a mixture of 2-thienylboronic acid pinacol ester, Pd₂(dba)₃ [tris(dibenzylideneacetone)-di-palladium)] and tri-tert-butyl-phosphonium-tetrafluoroborate in tetrahydrofurane.

The 2-thienylboronic acid pinacol ester may be obtained e.g. by adding substituted or unsubstituted thiophene to a mixture prepared from n-butyl-lithium and diisopropylamine and by adding 2-isopropoxy-4,4,5,5-tetramethyl-1,3,2-dioxaborolane to the thus obtained mixture.

Analogously, the "side-chain-elongation" of the compounds of the formulae **XLII** or **XLIII** with an additional phenyl or biphenyl residue may be effected with phenyl-boronic acid pinacol ester or biphenyl-boronic acid pinacol ester.

Alternatively, for the manufacture of compounds of the formula 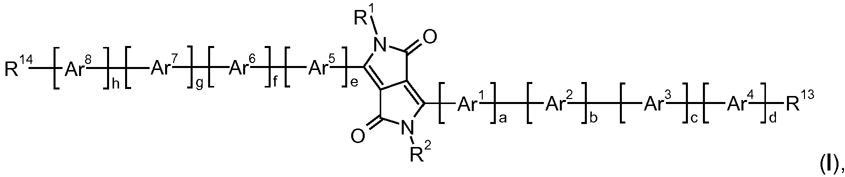 -wherein the side chains of the formulae 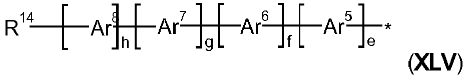 and 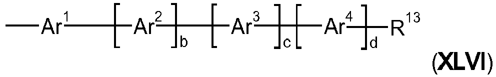 are identical to each other and R¹³ and R¹⁴ are H, it is also possible to build up the complete side chains first and then reacting a nitrile of the formula 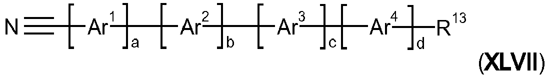 with a suitable disuccinate, e.g. di-tert-amyl succinate. For example, a mixture of iron(III)chloride (FeCl₃), sodium, and tert-amylalcohol may be heated to 60-160°C, e.g. 110 °C, before a mixture of the nitrile of the formula **XLVII** and di-tert-amyl succinate is added drop wise. After stirring the reaction mixture until the reaction is complete, e.g. for about 19 hours at 110 °C, the reaction mixture is poured onto a water-methanol mixture.

Compounds of the formulae 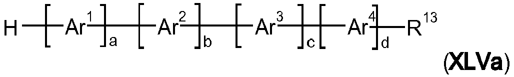 and 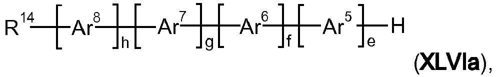 wherein R¹³ and R¹⁴ are H, containing the complete side chains can be manufactured e.g. by reacting a bromo derivative of the formula Br-Ar¹ etc. first with magnesium in diethyl ether and then adding the thus obtained Grignard solution to a solution in diethyl ether of Ni(dppp)Cl₂ and a mono- or, if desired, dibromo compound of the formula Br-Ar² or Br-Ar²-Br, respectively, etc.

The conversion of a compound of the formula **XLVIa** into the nitrile of the formula **XLVII** may be effected e.g. by adding a solution of a compound of the formula **XLVIa,** e.g. in toluene, to the reaction mixture obtained by adding triflic anhydride to a solution of N-formylmethylaniline in e.g. toluene, and reacting the obtained aldehyde of the formula 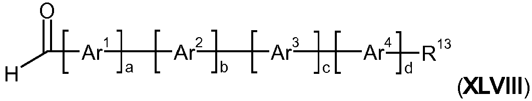 with hydroxylamine sulfate in e.g. dimethyl formamide.

The thus obtained compound of the formula I wherein R¹ and R² are hydrogen may then be transformed into a desired end product of the formula I wherein R¹ and R² are e.g. an aliphatic, cycloaliphatic, cycloaliphatic-aliphatic, or aromatic-aliphatic group, like especially such an hydrocarbon group, by N-alkylation, e.g. analogously as described above, or by heating a solution thereof and potassium carbonate in dimethyl formamide followed by addition of R¹-Br or R²-Br, or by reaction with a suitable iodide of the formula R¹-I or R²-I. For example, a mixture of a compound of the formula I wherein R¹ and R² are hydrogen in N-methyl-pyrrolidone is treated, preferably under cooling, e.g. to a temperature between about 0 °C and 10 °C, e.g. about 5 °C, with a suitable strong base, e.g. a suitable hydride, like an alkali metal hydride, e.g. sodium hydride. Thereafter, the iodide of the formula R¹-I or R²-I is added. R¹ and R² are preferably identical.

Halogen is fluorine, chlorine, bromine and iodine.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, octadecyl, eicosyl, heneicosyl, docosyl, tetracosyl or pentacosyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₂-C₂₅alkenyl groups are straight-chain or branched alkenyl groups, such as e.g. vinyl, allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-dodec-2-enyl or n-octadec-4-enyl.

C₂₋₂₅alkynyl is straight-chain or branched and preferably C₂₋₈alkynyl, which may be unsubstituted or substituted, such as, for example, ethynyl, 1-propyn-3-yl, 1-butyn-4-yl, 1-pentyn-5-yl, 2-methyl-3-butyn-2-yl, 1,4-pentadiyn-3-yl, 1,3-pentadiyn-5-yl, 1-hexyn-6-yl, cis-3-methyl-2-penten-4-yn-1-yl, trans-3-methyl-2-penten-4-yn-1-yl, 1,3-hexadiyn-5-yl, 1-octyn-8-yl, 1-nonyn-9-yl, 1-decyn-10-yl, or 1-tetracosyn-24-yl.

Aliphatic groups can, in contrast to aliphatic hydrocarbon groups, be substituted by any acyclic substituents, but are preferably unsubstituted. Preferred substituents are C₁-C₈alkoxy or C₁-C₈alkylthio groups as exemplified further below. The term "aliphatic group" comprises also alkyl groups wherein certain non-adjacent carbon atoms are replaced by oxygen, like -CH₂-O-CH₂-CH₂-O-CH₃. The latter group can be regarded as methyl substituted by -O-CH₂-CH₂-O-CH₃.

An aliphatic hydrocarbon group having up to 25 carbon atoms is a linear or branched alkyl, alkenyl or alkynyl (also spelled alkinyl) group having up to 25 carbon atoms as exemplified above.

Alkylene is bivalent alkyl, i.e. alkyl having two (instead of one) free valencies, e.g. trimethylene or tetramethylene.

Alkenylene is bivalent alkenyl, i.e. alkenyl having two (instead of one) free valencies, e.g. -CH₂-CH=CH-CH₂-.

Aliphatic groups can, in contrast to aliphatic hydrocarbon groups, be substituted by any acyclic substituents, but are preferably unsubstituted. Preferred substituents are C₁-C₈alkoxy or C₁-C₈alkylthio groups as exemplified further below. The term "aliphatic group" comprises also alkyl groups wherein certain non-adjacent carbon atoms are replaced by oxygen, like -CH₂-O-CH₂-CH₂-O-CH₃. The latter group can be regarded as methyl substituted by -O-CH₂-CH₂-O-CH₃.

A cycloaliphatic hydrocarbon group is a cycloalkyl or cycloalkenyl group which may be substituted by one or more aliphatic and/or cycloaliphatic hydrocarbon groups.

A cycloaliphatic-aliphatic group is an aliphatic group substituted by a cycloaliphatic group, wherein the terms "cycloaliphatic" and "aliphatic" have the meanings given herein and wherein the free valency extends from the aliphatic moiety. Hence, a cycloaliphatic-aliphatic group is for example a cycloalkyl-alkyl group.

A cycloalkyl-alkyl group is an alkyl group substituted by a cycloalkyl group, e.g. cyclohexyl-methyl.

A "cycloalkenyl group" means an unsaturated alicyclic hydrocarbon group containing one or more double bonds, such as cyclopentenyl, cyclopentadienyl, and cyclohexenyl, which may be unsubstituted or substituted by one or more aliphatic and/or cycloaliphatic hydrocarbon groups and/or condensed with phenyl groups.

For example, a cycloalkyl or cycloalkenyl group, in particular a cyclohexyl group, can be condensed one or two times with phenyl which can be substituted one to three times with C₁-C₄-alkyl. Examples of such condensed cyclohexyl groups are groups of the formulae: 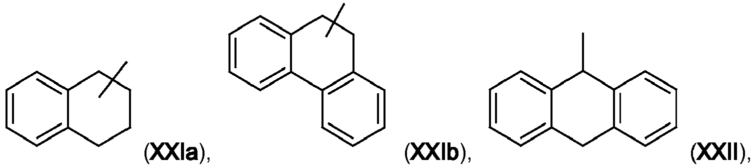 in particular 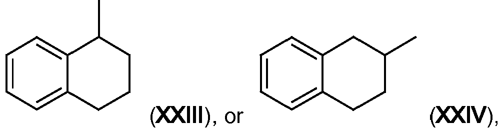 which can be substituted in the phenyl moieties one to three times with C₁-C₄-alkyl.

A bivalent group of the formula **XII** wherein R²⁸ and R²⁷ together represent alkylene or alkenylene which may be both bonded via oxygen and/or sulfur to the thienyl residue and which may both have up to 25 carbon atoms, is e.g. a group of the formula 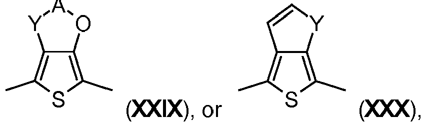 wherein A represents linear or branched alkylene having up to 25 carbon atoms, preferably ethylene or propylene which may be substituted by one or more alkyl groups, and Y represents oxygen or sulphur. For example, the bivalent group of the formula -Y-A-O- represents -O-CH₂-CH₂-O- or -O-CH₂-CH₂-CH₂-O-.

A group of the formula XI wherein two groups R²² to R²⁶ which are in the neighborhood of each other, together represent alkylene or alkenylene having up to 8 carbon atoms, thereby forming a ring, is e.g. a group of the formula 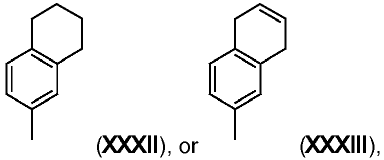 wherein in the group of the formula **XXXII** R²³ and R²⁴ together represent 1,4-butylene and in the group of the formula **XXXIII** R²³ and R²⁴ together represent 1,4-but-2-en-ylene.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentoxy, 2-pentoxy, 3-pentoxy, 2,2-dimethylpropoxy, n-hexoxy, n-heptoxy, n-octoxy, 1,1,3,3-tetramethylbutoxy and 2-ethylhexoxy, preferably C₁-C₄alkoxy such as typically methoxy, ethoxy, n-propoxy, isopropoxy, n-butoxy, sec.-butoxy, isobutoxy, tert.-butoxy. The term "alkylthio group" means the same groups as the alkoxy groups, except that the oxygen atom of the ether linkage is replaced by a sulfur atom.

C₁-C₁₈perfluoroalkyl, especially C₁-C₄perfluoroalkyl, is a branched or unbranched radical such as for example -CF₃, -CF₂CF₃, -CF₂CF₂CF₃, -CF(CF₃)₂, -(CF₂)₃CF₃, and -C(CF₃)₃.

The term "carbamoyl group" is typically a C₁₋₁₈carbamoyl radical, preferably C₁₋₈carbamoyl radical, which may be unsubstituted or substituted, such as, for example, carbamoyl, methylcarbamoyl, ethylcarbamoyl, n-butylcarbamoyl, tert-butylcarbamoyl, dimethylcarbamoyloxy, morpholinocarbamoyl or pyrrolidinocarbamoyl.

A cycloalkyl group is typically C₅-C₁₂cycloalkyl, such as, for example, cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted. The cycloalkyl group, in particular a cyclohexyl group, can be condensed one or two times by phenyl which can be substituted one to three times with C₁-C₄-alkyl, halogen and cyano. Examples of such condensed cyclohexyl groups are: 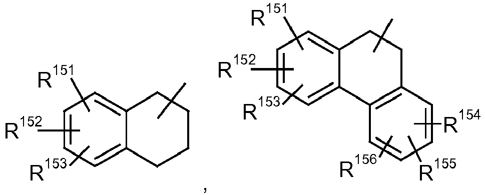 or 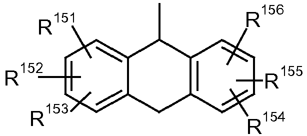 in particular 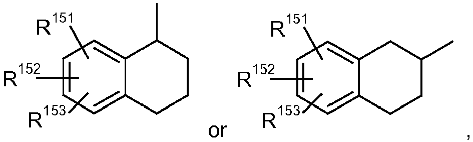 wherein R¹⁵¹, R¹⁵², R¹⁵³, R¹⁵⁴, R¹⁵⁵ and R¹⁵⁶ are independently of each other C₁-C₈-alkyl, C₁-C₈-alkoxy, halogen and cyano, in particular hydrogen.

C₆-C₂₄aryl (C₆-C₁₈aryl) is typically phenyl, indenyl, azulenyl, naphthyl, biphenyl, as-indacenyl, s-indacenyl, acenaphthylenyl, fluorenyl, phenanthryl, fluoranthenyl, triphenlenyl, chrysenyl, naphthacen, picenyl, perylenyl, pentaphenyl, hexacenyl, pyrenyl, or anthracenyl, preferably phenyl, 1-naphthyl, 2-naphthyl, 4-biphenyl, 9-phenanthryl, 2- or 9-fluorenyl, 3- or 4-biphenyl, which may be unsubstituted or substituted. Examples of C₆-C₁₂aryl are phenyl, 1-naphthyl, 2-naphthyl, 3- or 4-biphenyl, 2- or 9-fluorenyl or 9-phenanthryl, which may be unsubstituted or substituted.

C₇-C₂₅aralkyl is typically benzyl, 2-benzyl-2-propyl, β-phenyl-ethyl, α,α-dimethytbenzyl, ω-phenyl-butyl, ω,ω-dimethyl-ω-phenyl-butyl, co-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethytbenzyl, ω-phenyl-butyl, co,co-dimethyl-co-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethytbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

Heteroaryl is typically C₂-C₂₀heteroaryl, i.e. a ring with five to seven ring atoms or a condensed ring system, wherein nitrogen, oxygen or sulfur are the possible hetero atoms, and is typically an unsaturated heterocyclic group with five to 30 atoms having at least six conjugated π-etectrons such as thienyl, benzo[b]thienyl, dibenzo[b,d]thienyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl or phenoxazinyl, which can be unsubstituted or substituted.

Possible substituents of the above-mentioned groups are C₁-C₈alkyl, a hydroxyl group, a mercapto group, C₁-C₈alkoxy, C₁-C₈alkylthio, halogen, halo-C₁-C₈alkyl, a cyano group, a carbamoyl group, a nitro group or a silyl group, especially C₁-C₈alkyl, C₁-C₈alkoxy, C₁-C₈alkylthio, halogen, halo-C₁-C₈alkyl, or a cyano group.

C₁-C₁₈alkyl interrupted by one or more O is, for example, (CH₂CH₂O)₁₋₉-R^{x}, where R^{x} is H or C₁-C₁₀alkyl, CH₂-CH(OR^{y'})-CH₂-O-R^{y}, where R^{y} is C₁-C₁₈alkyl, and R^{y'} embraces the same definitions as R^{y} or is H.

If a substituent, such as, for example R³, occurs more than one time in a group, it can be different in each occurrence.

The compounds of the **(Ia)**, **(Ib)**, **(Ic),** or **(Id)** can show p-type transistor behavior and can be used as the semiconductor layer in semiconductor devices. Accordingly, the present invention also relates to a semiconductor device comprising as a semiconducting effective means a compound of the **(Ia)**, **(Ib)**, **(Ic),** or **(Id).**

The invention relates especially to a semiconductor device comprising as as a semiconducting effective means a compound of the **(Ia)**, **(Ib)**, (Ic), or **(Id)** described in the Examples selected from the compounds having the formulae **A-1** to **A-19**, respectively, which are depicted in claim 10.

Preferably said semiconductor device is a diode, a photodiode, a sensor, an organic field effect transistor (OFET), a transistor for flexible displays, or a solar cell, or a device containing a diode and/or an organic field effect transistor, and/or a solar cell. There are numerous types of semiconductor devices. Common to all is the presence of one or more semiconductor materials. Semiconductor devices have been described, for example, by S. M. Sze in Physics of Semiconductor Devices, 2nd edition, John Wiley and Sons, New York (1981). Such devices include rectifiers, transistors (of which there are many types, including p-n-p, n-p-n, and thin-film transistors), light emitting semiconductor devices (for example, organic light emitting diodes in display applications or backlight in e.g. liquid crystal displays), photoconductors, current limiters, solar cells, thermistors, p-n junctions, field-effect diodes, Schottky diodes, and so forth. In each semiconductor device, the semiconductor material is combined with one or more metals and/or insulators to form the device. Semiconductor devices can be prepared or manufactured by known methods such as, for example, those described by Peter Van Zant in Microchip Fabrication, Fourth Edition, McGraw-Hill, New York (2000). In particular, organic electronic components can be manufactured as described by D.R. Gamota et al. in Printed Organic and Molecular Electronics, Kluver Academic Publ., Boston, 2004.

A particularly useful type of transistor device, the thin-film transistor (TFT), generally includes a gate electrode, a gate dielectric on the gate electrode, a source electrode and a drain electrode adjacent to the gate dielectric, and a semiconductor layer adjacent to the gate dielectric and adjacent to the source and drain electrodes (see, for example, S. M. Sze, Physics of Semiconductor Devices, 2nd edition, John Wiley and Sons, page 492, New York (1981)). These components can be assembled in a variety of configurations. More specifically, an organic thin-film transistor (OTFT) has an organic semiconductor layer.

Typically, a substrate supports the OTFT during manufacturing, testing, and/or use. Optionally, the substrate can provide an electrical function for the OTFT. Useful substrate materials include organic and inorganic materials. For example, the substrate can comprise silicon materials inclusive of various appropriate forms of silicon, inorganic glasses, ceramic foils, polymeric materials (for example, acrylics, polyester, epoxies, polyamides, polycarbonates, polyimides, polyketones, poly(oxy-1,4-phenyleneoxy-1,4-phenylenecarbonyl-1,4-phenylene) (sometimes referred to as poly(ether ether ketone) or PEEK), polynorbornenes, polyphenyleneoxides, poly(ethylene naphthalenedicarboxylate) (PEN), poly(ethylene terephthalate) (PET), poly(phenylene sulfide) (PPS)), filled polymeric materials (for example, fiber-reinforced plastics (FRP)), and coated metallic foils.

The gate electrode can be any useful conductive material. For example, the gate electrode can comprise doped silicon, or a metal, such as aluminum, chromium, gold, silver, nickel, palladium, platinum, tantalum, and titanium. Conductive oxides, such as indium tin oxide (ITO), or conducting inks/pastes comprised of carbon black/graphite or colloidal silver dispersions, optionally containing polymer binders can also be used. Conductive polymers also can be used, for example polyaniline or poly(3,4-ethylenedioxythiophene)/poly(styrene sulfonate) (PEDOT:PSS). In addition, alloys, combinations, and multilayers of these materials can be useful. In some OTFTs, the same material can provide the gate electrode function and also provide the support function of the substrate. For example, doped silicon can function as the gate electrode and support the OTFT.

The gate dielectric is generally provided on the gate electrode. This gate dielectric electrically insulates the gate electrode from the balance of the OTFT device. Useful materials for the gate dielectric can comprise, for example, an inorganic electrically insulating material.

The gate dielectric (insulator) can be a material, such as, an oxide, nitride, or it can be a material selected from the family of ferroelectric insulators (e.g. organic materials such as poly(vinylidene fluoride/trifluoroethylene or poly(m-xylylene adipamide)), or it can be an organic polymeric insulator (e.g. poly(methacrylate)s, poly(acrylate)s, polyimides, benzocyclobutenes (BCBs), parylenes, polyvinylalcohol, polyvinylphenol (PVP), polystyrenes, polyester, polycarbonates) as for example described in J. Veres et al. Chem. Mat. 2004, 16, 4543 or A. Facchetti et al. Adv. Mat. 2005, 17, 1705. Specific examples of materials useful for the gate dielectric include strontiates, tantalates, titanates, zirconates, aluminum oxides, silicon oxides, tantalum oxides, titanium oxides, silicon nitrides, barium titanate, barium strontium titanate, barium zirconate titanate, zinc selenide, and zinc sulphide, including but not limited to PbZrₓTi₁₋ₓO₃ (PZT), Bi₄Ti₃O₁₂, BaMgF₄, Ba(Zr₁₋ₓTiₓ)O₃ (BZT). In addition, alloys, hybride materials (e.g. polysiloxanes or nanoparticle-filled polymers) combinations, and multilayers of these materials can be used for the gate dielectric. The thickness of the dielectric layer is, for example, from about 10 to 1000 nm, with a more specific thickness being about 100 to 500 nm, providing a capacitance in the range of 0.1 - 100 nanofarads (nF).

The source electrode and drain electrode are separated from the gate electrode by the gate dielectric, while the organic semiconductor layer can be over or under the source electrode and drain electrode. The source and drain electrodes can be any useful conductive material favourably providing a low resistance ohmic contact to the semiconductor layer. Useful materials include most of those materials described above for the gate electrode, for example, aluminum, barium, calcium, chromium, gold, silver, nickel, palladium, platinum, titanium, polyaniline, PEDOT:PSS, other conducting polymers, alloys thereof, combinations thereof, and multilayers thereof. Some of these materials are appropriate for use with n-type semiconductor materials and others are appropriate for use with p-type semiconductor materials, as is known in the art.

The thin film electrodes (that is, the gate electrode, the source electrode, and the drain electrode) can be provided by any useful means such as physical vapor deposition (for example, thermal evaporation or sputtering) or (ink jet) printing methods. The patterning of these electrodes can be accomplished by known methods such as shadow masking, additive photolithography, subtractive photolithography, printing, microcontact printing, and pattern coating.

The present invention further provides a thin film transistor device comprising
a plurality of electrically conducting gate electrodes disposed on a substrate;
a gate insulator layer disposed on said electrically conducting gate electrodes;
a plurality of sets of electrically conductive source and drain electrodes disposed on said insulator layer such that each of said sets is in alignment with each of said gate electrodes;
an organic semiconductor layer disposed in the channel between source and drain electrodes on said insulator layer substantially overlapping said gate electrodes; wherein said organic semiconductor layer comprise a compound of the **(Ia)**, **(Ib)**, **(Ic),** or **(Id).**

An organic field effect transistor can have various designs. The most common design of a field-effect transistor is the Bottom-Gate Top-Contact (BGTC) design. Here, the gate is on top of the substrate and at the bottom of the dielectric layer, the semiconducting layer is at the top of the dielectric layer and the source/drain electrodes are on top of the semiconducting layer.

Another design of a field-effect transistor is the Top-Gate Bottom-Contact (TGBC) design. Here, the source/drain electrodes are on top of the substrate and at the bottom of the semiconducting layer, the dielectric layer is on top of the semiconducting layer and the gate electrode is on top of the dielectric layer.

The organic semiconductor layer is preferably formed by applying a solution and/or dispersion of a compound according to the present invention in an organic solvent to a suitable substrate and removing the solvent; or the organic semiconductor layer is formed by vacuum vapor deposition of a compound according to the present invention.

The present invention further provides a process for preparing a thin film transistor device comprising the steps of:
depositing a plurality of electrically conducting gate electrodes on a substrate;
depositing a gate insulator layer on said electrically conducting gate electrodes;
depositing a plurality of sets of electrically conductive source and drain electrodes on said layer such that each of said sets is in alignment with each of said gate electrodes;
depositing a layer comprising a compound of the **(Ia)**, **(Ib)**, **(Ic),** or **(Id)** on said insulator layer such that said layer comprising the compound of **(Ia)**, **(Ib)**, **(Ic),** or **(Id)** substantially overlaps said gate electrodes, thereby producing the thin film transistor device.

The above-mentioned layer comprising a compound of **(Ia)**, **(Ib)**, **(Ic),** or **(Id)** may additionally comprise at least another material. The other material can be, but is not restricted to another compound of the **(Ia)**, **(Ib)**, **(Ic),** or **(Id),** a semi-conducting polymer, a polymeric binder, organic small molecules different from a compound of the **(Ia)**, **(Ib)**, **(Ic),** or **(Id),** carbon nanotubes, a fullerene derivative, inorganic particles (quantum dots, quantum rods, quantum tripods, TiO₂, ZnO etc.), conductive particles (Au, Ag etc.), and insulator materials like the ones described for the gate dielectric (PET, PS etc.). As stated above, the semiconductive layer can also be composed of a mixture of one or more small molecules of the **(Ia)**, **(Ib)**, **(Ic),** or **(Id)** and a polymeric binder. The ratio of the small molecules of **(Ia)**, **(Ib)**, **(Ic),** or **(Id)** to the polymeric binder can vary from 5 to 95 percent. Preferably, the polymeric binder is a semicristalline polymer such as polystyrene (PS), high-density polyethylene (HDPE), polypropylene (PP) and polymethylmethacrylate (PMMA). With this technique, a degradation of the electrical performance can be avoided (cf. WO 2008/001123 A1).

Any suitable substrate can be used to prepare the thin films of the compounds of the **(Ia)**, **(Ib)**, (Ic), or **(Id).** Preferably, the substrate used to prepare the above thin films is a metal, silicon, plastic, paper, coated paper, fabric, glass or coated glass.

In a preferred embodiment, the deposition of at least one compound of the general **(Ia)**, **(Ib)**, **(Ic),** or **(Id)** (and if appropriate further semiconductor materials) is carried out by a gas phase deposition process (physical vapor deposition, PVD). PVD processes are performed under high-vacuum conditions and comprise the following steps: evaporation, transport, deposition. It has been found that, surprisingly, the compounds of the general **(Ia)**, **(Ib)**, **(Ic),** or **(Id)** are suitable particularly advantageously for use in a PVD process, since they essentially do not decompose and/or form undesired by-products. The material deposited is obtained in high purity. In a specific embodiment, the deposited material is obtained in the form of crystals or comprises a high crystalline content. In general, for the PVD, at least one compound of the general **(Ia)**, **(Ib)**, **(Ic),** or **(Id)** is heated to a temperature above its evaporation temperature and deposited on a substrate by cooling below the crystallization temperature. The temperature of the substrate in the deposition is preferably within a range from about 20 to 250°C, more preferably from 50 to 200°C.

The resulting semiconductor layers generally have a thickness which is sufficient for ohmic contact between source and drain electrodes. The deposition can be effected under an inert atmosphere, for example under nitrogen, argon or helium. The deposition is effected typically at ambient pressure or under reduced pressure. A suitable pressure range is from about 10⁻⁷ to 1.5 bar.

The compound of the **(Ia)**, **(Ib)**, **(Ic),** or **(Id)** is preferably deposited on the substrate in a thickness of from 10 to 1000 nm, more preferably from 15 to 250 nm. In a specific embodiment, the compound of the **(Ia)**, **(Ib)**, **(Ic),** or **(Id)** is deposited at least partly in crystalline form. For this purpose, especially the above-described PVD process is suitable. Moreover, it is possible to use previously prepared organic semiconductor crystals. Suitable processes for obtaining such crystals are described by R.A. Laudise et al. in "Physical Vapor Growth of Organic SemiConductors", Journal of Crystal Growth 187 (1998), pages 449-454, and in "Physical Vapor Growth of Centimeter-sized Crystals of α-Hexathiophene", Journal of Crystal Growth 1982 (1997), pages 416-427.

Alternatively, a TFT is fabricated, for example, by solution deposition of a compound of the **(Ia)**, **(Ib)**, **(Ic),** or **(Id)** on a highly doped silicon substrate covered with a thermally grown oxide layer followed by vacuum deposition and patterning of source and drain electrodes.

In yet another approach, a TFT is fabricated by deposition of source and drain electrodes on a highly doped silicon substrate covered with a thermally grown oxide and then solution deposition of the compound of the **(Ia)**, **(Ib)**, **(Ic),** or **(Id)** to form a thin film.

The gate electrode could also be a patterned metal gate electrode on a substrate or a conducting material such as a conducting polymer, which is then coated with an insulator applied either by solution coating or by vacuum deposition on the patterned gate electrodes.

Self-assembled monolayer (SAMs) interposed between a gate dielectric and an organic semiconductor layer have been utilised to create more compatible interfaces. Early examples of using SAMs included using a silazane or silane coupling agents on silicon oxide surfaces. Other approaches have been to include a polymeric interlayer between the dielectric and semiconductor layers; or alkyl phosphonic acid, or fluoro alkyl phosphonic acid SAMs (see, for example, U. Kraft et al., J. Mater. Chem. 20 (2010) 6416-18).

Any suitable solvent can be used to dissolve, and/or disperse a compound of the **(Ia)**, **(Ib)**, **(Ic),** or **(Id),** provided it is inert and can be removed partly, or completely from the substrate by conventional drying means (e.g. application of heat, reduced pressure, airflow etc.). Suitable organic solvents for processing the semiconductors of the invention include, but are not limited to, aromatic or aliphatic hydrocarbons, halogenated such as chlorinated or fluorinated hydrocarbons, esters, ethers amides, such as chloroform, tetrachloroethane, tetrahydrofuran, toluene, tetraline, anisole, xylene, ethyl acetate, methyl ethyl ketone, dimethyl formamide, dichlorobenzene, trichlorobenzene, propylene glycol monomethyl ether acetate (PGMEA) and mixtures thereof. The solution, and/or dispersion is then applied by a method, such as, spin-coating, dip-coating, screen printing, microcontact printing, doctor blading or other solution application techniques known in the art on the substrate to obtain thin films of the semiconducting material.

The term "dispersion" covers any composition comprising a compound of the **(Ia)**, **(Ib)**, (Ic), or **(Id),** which is not fully dissolved in a solvent. The dispersion can be done selecting a composition including at least a compound of **(Ia)**, **(Ib)**, **(Ic),** or **(Id),** or a mixture containing a compound of **(Ia)**, **(Ib)**, (I**c**), or **(Id),** and a solvent, wherein the polymer exhibits lower solubility in the solvent at room temperature but exhibits greater solubility in the solvent at an elevated temperature, wherein the composition gels when the elevated temperature is lowered to a first lower temperature without agitation;
- dissolving at the elevated temperature at least a portion of the compound of the **(Ia)**, **(Ib)**, **(Ic),** or **(Id)** in the solvent; lowering the temperature of the composition from the elevated temperature to the first lower temperature; agitating the composition to disrupt any gelling, wherein the agitating commences at any time prior to, simultaneous with, or subsequent to the lowering the elevated temperature of the composition to the first lower temperature; depositing a layer of the composition wherein the composition is at a second lower temperature lower than the elevated temperature; and drying at least partially the layer.

The dispersion can also be constituted of (a) a continuous phase comprising a solvent, a binder resin, and optionally a dispersing agent, and (b) a disperse phase comprising a compound of **(Ia)**, **(Ib)**, **(Ic),** or **(Id),** or a mixture containing a compound of **(Ia)**, **(Ib)**, **(Ic),** or **(Id)** of the present invention. The degree of solubility of the compound of **(Ia)**, **(Ib)**, **(Ic),** or **(Id)** in the solvent may vary for example from 0.5 % to about 20 % solubility, particularly from 1 % to about 5 % solubility.

Preferably, the thickness of the organic semiconductor layer is in the range of from about 5 to about 1000 nm, especially the thickness is in the range of from about 10 to about 100 nm.

The compounds of the **(Ia)**, **(Ib)**, **(Ic),** or **(Id)** can be used alone or in combination as the organic semiconductor layer of the semiconductor device. The layer can be provided by any useful means, such as, for example, vapor deposition and printing techniques. The compounds of the **(Ia)**, **(Ib)**, (**Ic**), or **(Id)** which are sufficiently soluble in organic solvents can be solution deposited and patterned (for example, by spin coating, dip coating, ink jet printing, gravure printing, flexo printing, offset printing, screen printing, microcontact (wave)-printing, drop or zone casting, or other known techniques).

The compounds of the **(Ia)**, **(Ib)**, **(Ic),** or **(Id)** can be used in integrated circuits comprising a plurality of OTFTs, as well as in various electronic articles. Such articles include, for example, radiofrequency identification (RFID) tags, backplanes for flexible displays (for use in, for example, personal computers, cell phones, or handheld devices), smart cards, memory devices, sensors (e.g. light-, image-, bio-, chemo-, mechanical- or temperature sensors), especially photodiodes, or security devices. Due to its ambi-polarity the material can also be used in Organic Light Emitting Transistors (OLET).

In addition, the invention provides organic photovoltaic (PV) devices (solar cells) comprising a compound of the **(Ia)**, **(Ib)**, **(Ic),** or **(Id).**

The PV device comprise in this order:
(a) a cathode (electrode),
(b) optionally a transition layer, such as an alkali halogenide, especially lithium fluoride,
(c) a photoactive layer,
(d) optionally a smoothing layer,
(e) an anode (electrode),
(f) a substrate.

The photoactive layer comprises the compounds of the **(Ia)**, **(Ib)**, **(Ic),** or **(Id).** Preferably, the photoactive layer is made of a compound of the **(Ia)**, **(Ib)**, **(Ic),** or **(Id),** as an electron donor and an acceptor material, like a fullerene, particularly a functionalized fullerene PCBM, as an electron acceptor. As stated above, the photoactive layer may also contain a polymeric binder. The ratio of the small molecules of **(Ia)**, **(Ib)**, **(Ic),** or **(Id)** to the polymeric binder can vary from 5 to 95 percent. Preferably, the polymeric binder is a semicristalline polymer such as polystyrene (PS), high-density polyethylene (HDPE), polypropylene (PP) and polymethylmethacrylate (PMMA).

The fullerenes useful in this invention may have a broad range of sizes (number of carbon atoms per molecule). The term fullerene as used herein includes various cage-like molecules of pure carbon, including Buckminsterfullerene (C₆₀) and the related "spherical" fullerenes as well as carbon nanotubes. Fullerenes may be selected from those known in the art ranging from, for example, C₂₀-C₁₀₀₀. Preferably, the fullerene is selected from the range of C₆₀ to C₉₆. Most preferably the fullerene is C₆₀ or C₇₀, such as [60]PCBM, or [70]PCBM. It is also permissible to utilize chemically modified fullerenes, provided that the modified fullerene retains acceptor-type and electron mobility characteristics. The acceptor material can also be a material selected from the group consisting of another polymer of **(Ia)**, **(Ib)**, **(Ic),** or **(Id)** or any semi-conducting polymer provided that the polymers retain acceptor-type and electron mobility characteristics, organic small molecules, carbon nanotubes, inorganic particles (quantum dots, quantum rods, quantum tripods, TiO₂, ZnO etc.).

For heterojunction solar cells (bulk heterojunction solar cells) the active layer comprises preferably a mixture of a compound of the **(Ia)**, **(Ib)**, **(Ic),** or **(Id)** and a fullerene, such as [60]PCBM (= 6,6-phenyl-C₆₁-butyric acid methyl ester), or [70]PCBM, in a weight ratio of 1:1 to 1:3. meth-anofullerene (phenyl-C₆₁-butyric-acid-methyl-ester ([60]PCBM), i.e. 1-[3-(methoxycarbonyl)propyl]-1-phenyl-[6.6]C₆₁-3'H-cyclopropa[1,9][5,6]fullerene-C₆₀-lh-3'-butanoic acid 3'-phenyl methyl ester, is an effective solution processable n-type organic semiconductor. It is blended with conjugated polymers with nano-particles such as C₆₀.

The electrodes are preferably composed of metals or "metal substitutes". Herein the term "metal" is used to embrace both materials composed of an elementally pure metal, e.g., Mg, and also metal alloys which are materials composed of two or more elementally pure metals, e.g., Mg and Ag together, denoted Mg:Ag. Here, the term "metal substitute" refers to a material that is not a metal within the normal definition, but which has the metal-like properties that are desired in certain appropriate applications. Commonly used metal substitutes for electrodes and charge transfer layers would include doped wide-bandgap semiconductors, for example, transparent conducting oxides such as indium tin oxide (ITO), gallium indium tin oxide (GITO), and zinc indium tin oxide (ZITO). Another suitable metal substitute is the transparent conductive polymer polyanaline (PANI) and its chemical relatives, or PEDOT:PSS. Metal substitutes may be further selected from a wide range of non-metallic materials, wherein the term "non-metallic" is meant to embrace a wide range of materials provided that the material is free of metal in its chemically uncombined form. Highly transparent, non-metallic, low resistance cathodes or highly efficient, low resistance metallic/non-metallic compound cathodes are, for example, disclosed in US-B-6,420,031 and US-B-5,703,436.

The substrate can be, for example, a plastic (flexible substrate), or glass substrate.

In another preferred embodiment of the invention, a smoothing layer is situated between the anode and the photoactive layer. A preferred material for this smoothing layer comprises a film of 3,4-polyethylenedioxythiophene (PEDOT), or 3,4-polyethylenedioxythiophene:polystyrene-sulfonate (PEDOT:PSS).

In a preferred embodiment of the present invention, the photovoltaic cell comprises, as described for example, in US-B-6,933,436 a transparent glass carrier, onto which an electrode layer made of indium/tin oxide (ITO) is applied. This electrode layer generally has a comparatively rough surface structure, so that it is covered with a smoothing layer made of a polymer, typically PEDOT, which is made electrically conductive through doping. The photoactive layer is made of two components, has a layer thickness of, for example, 100 nm to a few µm depending on the application method, and is applied onto this smoothing layer. The photoactive layer is made of a compound of the **(Ia)**, **(Ib)**, (Ic), or (Id), as an electron donor and a fullerene, particularly functionalized fullerene PCBM, as an electron acceptor. These two components are mixed with a solvent and applied as a solution onto the smoothing layer by, for example, the spin-coating method, the drop casting method, the Langmuir-Blodgett ("LB") method, the ink jet printing method and the dripping method. A squeegee or printing method could also be used to coat larger surfaces with such a photoactive layer. Instead of toluene, which is typical, a dispersion agent such as chlorobenzene is preferably used as a solvent. Among these methods, the vacuum deposition method, the spin-coating method, the ink jet printing method and the casting method are particularly preferred in view of ease of operation and cost.

In the case of forming the layer by using the spin-coating method, the casting method and ink jet printing method, the coating can be carried out using a solution and/or dispersion prepared by dissolving, or dispersing the composition in a concentration of from 0.01 to 90% by weight in an appropriate organic solvent such as benzene, toluene, xylene, tetrahydrofurane, methyltetrahy-drofurane, N,N-dimethylformamide, acetone, acetonitrile, anisole, dichloromethane, dimethylsulfoxide, chlorobenzene, 1,2-dichlorobenzene and mixtures thereof.

Before a counter electrode is applied, a thin transition layer, which must be electrically insulating, having a layer thickness of, for example, 0.6 nm, is applied to the photoactive layer. In this exemplary embodiment, this transition layer is made of an alkali halogenide, namely a lithium fluoride, which is vapor deposited in a vacuum of 2 · 10⁻⁶ torr at a rate of 0.2 nm/minute.

If ITO is used as a hole-collecting electrode, aluminum, which is vapor deposited onto the electrically insulating transition layer, is used as an electron-collecting electrode. The electric insulation properties of the transition layer obviously prevent influences which hinder the crossing of the charge carrier from being effective, particularly in the transition region from the photoactive layer to the transition layer.

In a further embodiment of the invention, one or more of the layers may be treated with plasma prior to depositing the next layer. It is particularly advantageous that prior to the deposition of the PEDOT:PSS layer the anode material is subjected to a mild plasma treatment.

As an alternative to PEDOT:PSS a crosslinkable hole-transport material based on triarylamines as referenced in Macromol. Rapid Commun. 20, 224-228 (1999) can be used. In addition to the triarylamine material the layer can also include an electron acceptor to improve electron transport. Such compounds are disclosed in US 2004/0004433. Preferably, the electron acceptor material is soluble in one or more organic solvents. Typically, the electron acceptor material is present in the range fo 0.5 to 20 % by weight of the triarylamine material.

The photovoltaic (PV) device can also consist of multiple junction solar cells that are processed on top of each other in order to absorb more of the solar spectrum. Such structures are, for example, described in App. Phys. Let. 90, 143512 (2007), Adv. Funct. Mater. 16, 1897-1903 (2006) and WO2004/112161.

A so called 'tandem solar cell' comprise in this order:
(a) a cathode (electrode),
(b) optionally a transition layer, such as an alkali halogenide, especially lithium fluoride,
(c) a photoactive layer,
(d) optionally a smoothing layer,
(e) a middle electrode (such as Au, Al, ZnO, TiO₂ etc.)
(f) optionally an extra electrode to match the energy level,
(g) optionally a transition layer, such as an alkali halogenide, especially lithium fluoride,
(h) a photoactive layer,
(i) optionally a smoothing layer,
(j) an anode (electrode),
(k) a substrate.

The PV device can also be processed on a fiber as described, for example, in US20070079867 and US 20060013549.

Due to their excellent self-organising properties the materials or films comprising the compounds of the **(Ia)**, **(Ib)**, **(Ic),** or **(Id)** can also be used alone or together with other materials in or as alignment layers in LCD or OLED devices, as described for example in US2003/0021913.

Various features and aspects of the present invention are illustrated further in the examples that follow. While these examples are presented to show one skilled in the art how to operate within the scope of this invention, they are not to serve as a limitation on the scope of the invention where such scope is only defined in the claims. Unless otherwise indicated in the following examples and elsewhere in the specification and claims, all parts and percentages are by weight, temperatures are in degrees centigrade and pressures are at or near atmospheric.

### Examples

### Materials and Methods

2,5-Di(2-ethylhexyl)-3,6-bis(5-bromo-thiophen-2-yl)pyrrolo[3,4-c]-pyrrole-1,4-dione 2 is prepared according to the literature (Tamayo, A. B.; Tantiwiwat, M.; Walker, B.; Nguyen, T.-Q. J. Phys. Chem. C 2008, 112, 15543-15552. (b) Huo, L.; Hou, J.; Chen, H.-Y.; Zhang, S.; Jiang, Y.; Chen, T. L.; Yang, Y.. Macromolecules 2009, 42, 6564-6571).

All other reagents and solvents are obtained from commercial suppliers and purified and dried according to standard procedures (Perrin, D. D.; Armarego, W. L.; Perrin, D. R. Purification of Laboratory Chemicals - 2nd ed.; Pergamon Press Ltd.: Oxford 1980). Column chromatography is performed on silica gel (Merck Silica 60, particle size 0.040-0.063 mm). Solvents for spectroscopic studies are of spectroscopic grade and used as received. Elemental analysis is performed on a CHNS 932 analyzer (Leco Instruments GmbH, Mönchengladbach, Germany). ¹H spectra are recorded in CD₂Cl₂ on a Bruker Avance 400 spectrometer. Residual undeuterated solvent is used as internal standard (5.32 ppm for ¹H). High-resolution ESI-TOF mass spectrometry is carried out on a microTOF focus instrument (Bruker Daltronik GmbH). UV/Vis measurements are performed in a conventional quartz cell (light pass 10 mm) on a Perkin-Elmer Lambda 950 spectrometer. For cyclic voltammetry, a standard commercial electrochemical analyzer (EC epsilon; BAS Instruments, UK) with a three electrode single-compartment cell is used. Dichloromethane (HPLC grade) is dried over calcium hydride under argon and degassed before using. The supporting electrolyte tetrabutylammonium hexafluorophosphate (TBAHFP) is prepared according to the literature (Fry, A. J. Laboratory Techniques in Electroanalytical Chemistry 2nd ed.; Marcel Dekker Ltd.: New York 1996), and recrystallized from ethanol/water. The measurements are carried out in dichloromethane at a concentration of about 10⁻⁴M with ferrocene (Fc) as internal standard for the calibration of the potential. Ag/AgCl reference electrode is used. A Pt disc and a Pt wire are used as working and auxiliary electrodes, respectively.

### Example 1 - 2,5-Di(2-ethylhexy))-3,6-bis(5-cyano-thiophen-2-y))pyirolo[3,4-c]-pyrrole-1,4-dione (A-1)

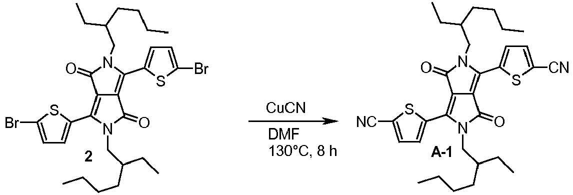

A mixture of compound 2 (249 mg, 0.365 mmol) and copper(I)cyanide (1.50 g, 16.7 mmol) in dry DMF (8 mL) is heated under argon for 8 h at 130 °C. The reaction mixture is allowed to cool down to room temperature and a solution of sodium cyanide (180 g) in water (20 mL) is added. The aqueous phase is extracted with dichloromethane. The solvent is removed under reduced pressure and the residue is purified by column chromatography (dichloromethane) affording a dark violet solid (48.8 mg, 31%).
**¹H NMR** (400 MHz, CD₂Cl₂): *δ* = 8.82 (d, ³*J*= 4.2 Hz, 2H), 7.76 (d, ³*J*= 4.2 Hz, 2H), 4.03-3.91 (m, 4H), 1.84-1.72 (m, 2H), 1.40-1.18 (m, 16H), 0.91-0.82 (m, 12H).
**¹³C NMR** (101 MHz, CD₂Cl₂): *δ* = 161.5, 139.8, 138.3, 135.7, 134.8, 113.9, 113.7, 110.9, 46.4, 39.7, 30.5, 28.7, 23.9, 23,4, 14.2, 10.5.
**HRMS** (ESI, acetonitrile/CHCl₃ 1:1, pos. mode): Calcd. for [M+H] C₃₂H₃₉N₄O₂S₂ 575.2510. Found 575.2510.
**Elemental Anal.** Calcd. for C₃₂H₃₈N₄O₂S₂: C, 66.87; H, 6.66; N, 9.75; S, 11.16; O, 3.94. Found: C, 66.60; H, 6.62; N, 9.77; S, 11.09.
**CV** (CH₂Cl₂, 0.1 M TBAHFP, vs. Fc/Fc⁺): *E*_{*1*/*2*}^{red} (X-/X²⁻) = -1.77 V, *E*_{*1*/*2*}^{red} (X/X-) = -1.24 V, *E*_{*1*/*2*}^{ox} (X/X+) = 0.86 V.
**UV-vis** (CH₂Cl₂): *λ*ₘₐₓ (ε) = 586 (28500), 546 nm (26500 M⁻¹ cm⁻¹).

The redox behavior is studied by cyclic voltammetry in dichloromethane with ferrocene as internal standard showing two reversible reductions at -1.24 V and -1.77 V as well as one reversible oxidation at 0.86 V. The optical absorption in dichloromethane shows a maximum at 586 nm. The optical band gap is determined as 2.12 eV from the absorption maximum.

### Application Example 1

To investigate the behavior of compound **A-1** in an active layer of a field effect transistor, bottom-gate top-contact thin film transistors (TFT) with a 110 nm thick SiO₂/AlOₓ/SAM gate dielectric are fabricated and measured in air. The organic compound is vacuum deposited at four different temperatures obtaining an optimum at 70°C (Table 1). Figure 1 shows the output and transfer characteristic of the TFT prepared under these conditions, leading to a hole mobility of 0.71 cm² V⁻¹ s⁻¹ determined in the saturation regime. In addition the device shows a high current on/off ratio (Iₒₙ/I_{off}) of 10⁶.

**Table 1: Summary of the performance for TFTs with compound A-1 fabricated at different deposition temperatures T_{D}.**

| T_{D} / °C | µ / cm² V⁻¹ s⁻¹ | Iₒₙ/ I_{off} | U_{T} / V |
|---|---|---|---|
| 20 | 0.2 | 10⁶ | -14 |
| 50 | 0.5 | 10⁶ | -19 |
| 70 | 0.7 | 10⁶ | -27 |
| 90 | 0.6 | 10⁶ | -25 |

The dependence of the mobility on the deposition temperature T_{D} shows an increase of the mobility from 20 °C to 50 °C reaching a maximum at 70 °C, whereas a slight decrease is obtained at 90 °C. To correlate these observations with the morphology of the organic layer, the latter is investigated by atomic force microscopy (AFM). With increasing the deposition temperature, the organic compound forms larger grains and thus the number of crystalline domains decreases. A lower number of grain boundaries should be favorable for the charge transport and this leads to an increase of the field-effect mobility.

In summary, the novel type of efficient p-channel material, compound **A-1,** exhibits high hole-mobilities and on/off-ratios in vacuum deposited TFTs.

### Example 2

### 2,5-Di-n-butyl-3,6-bis(5-cyano-thiophen-2-yl)pyrrolo[3,4-c]-pyrrole-1,4-dione (A-2)

The synthesis of 2,5-di-*n*-butyl-3,6-bis(5-bromo-thiophen-2-yl)pyrrolo[3,4-c]-pyrrole-1,4-dione is descrribed in Zhou et al., Macromolecules 43 (2010) 821-826.

2,5-Di-*n*-butyl-3,6-bis(5-bromo-thiophen-2-yl)pyrrolo[3,4-c]-pyrrole-1,4-dione (1.08 g, 1.89 mmol) and copper(I)-cyanide (4.00 g, 44.6 mmol) in dry DMF (15 mL) are heated under argon at 130 °C for 315 min and at 165 °C for 40 min. The solvent is removed under reduced pressure and the crude product continuously extracted from the reaction mixture with chloroform. The extract is purified by column chromatography (dichloromethane) and washed with hexane affording 333 mg (38%) of a dark violet solid.

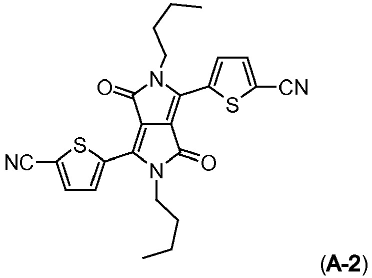

**¹H NMR** (400 MHz, CD₂Cl₂): δ 8.87 (d, ³*J*= 4.2 Hz, 2H), 7.77 (d, ³*J*= 4.2 Hz, 2H), 4.04 (t, ³*J*= 7.6 Hz), 1.74 - 1.64 (m, 4H), 1.49 - 1.38 (m, 4H), 0.97 (t, ³*J*= 7.4 Hz; 6H).
**HRMS** (ESI, pos-mode): calc. for [M-H]⁺ C₂₄H₂₃N₄O₂S₂: 463.1257, found: 463.1256.
**Elemental analysis:** calc. for C₂₄H₂₂N₄O₂S₂: C, 62.31; H, 4.79; N, 12.11; O, 6.92; S, 13.86; found: C, 62.32; H, 4.82; N, 12.05; S, 13.94.

### Application Example 2

To investigate the behavior of compound **A-2** in an active layer of a field effect transistor, bottom-gate top-contact thin film transistors (TFT) with a 110 nm thick SiO₂/AlOₓ/SAM gate dielectric are fabricated and measured in air. The organic compound is vacuum deposited at three different temperatures (Table 2).

**Table 2: Summary of the performance for TFTs with compound A-2 fabricated at different deposition temperatures T_{D} on SiO₂/AlOₓ/SAM with different self-assembled monolayers (SAMs).**

| T_{D} / °C | µ / cm² V⁻¹ s⁻¹ | µ / cm² V⁻¹ s⁻¹ |
|---|---|---|
| SAM | C₁₄H₂₉PO(OH)₂ | F₁₅C₁₈H₂₂PO(OH)₂ |
| 20 | 0.005 (p) | 0.01 (p) |
| | - (n) | - (n) |
| 50 | 0.03 (p) | |
| | 0.02 (n) | |
| 70 | 0.05 (p) | |
| | 0.06 (n) | |

### Example 3

### 2,5-Di-n-pentyl-3,6-bis(5-cyano-thiophen-2-yl)pyrrolo[3,4-c]-pyrrole-1,4-dione (A-17)

The synthesis of 2,5-di-*n*-pentyl-3,6-bis(5-bromo-thiophen-2-yl)pyrrolo[3,4-c]-pyrrole-1,4-dione is done in analogy to Zhou et al., Macromolecules 43 (2010) 821-826.

2,5-Di-*n*-pentyl-3,6-bis(5-bromo-thiophen-2-yl)pyrrolo[3,4-c]-pyrrole-1,4-dione (600 mg, 1.00 mmol) and copper(I)-cyanide (1.60 g, 18.0 mmol) in dry DMF (15 mL) are heated under argon at 120 °C for 60 min and at 165 °C for 80 min. The solvent is removed under reduced pressure and the crude product continuously extracted from the reaction mixture with chloroform. The extract is purified by column chromatography (dichloromethane) and washed with hexane affording 64.0 mg (13%) of a dark violet solid.

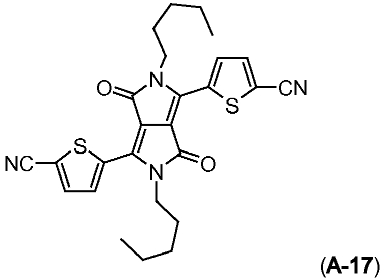

**¹H NMR** (400 MHz, CD₂Cl₂): δ 8.87 (d, ³*J*= 4.2 Hz, 2H), 7.77 (d, ³*J*= 4.2 Hz, 2H), 4.03 (t, ³*J*= 7.8 Hz), 1.75 - 1.66 (m, 4H), 1.44 - 1.34 (m, 8H), 0.92 (t, ³*J*= 7.1 Hz; 6H).
**HRMS** (ESI, pos-mode): calc. for [M+H] C₂₆H₂₇N₄O₂S₂: 491.1570, found: 491.1568.
**Elemental analysis:** calc. for C₂₆H₂₆N₄O₂S₂ C, 63.65; H, 5.34; N, 11.42; O, 6.52; S, 13.07, found C, 63.57; H, 5.23; N, 11.38; S, 13.05.

### Application Example 3

To investigate the behavior of compound **A-17** in an active layer of a field effect transistor, bottom-gate top-contact thin film transistors (TFT) with a 110 nm thick SiO₂/AlOₓ/SAM gate dielectric are fabricated and measured in air. The organic compound is vacuum deposited at two different temperatures (Table 3).

**Table 3: Summary of the performance for TFTs with compound A-17 fabricated at different deposition temperatures T_{D} on SiO₂/AlOₓ/SAM with different SAMs.**

| T_{D} / °C | µ / cm² V⁻¹ s⁻¹ | µ / cm² V⁻¹ s⁻¹ |
|---|---|---|
| SAM | C₁₄H₂₉PO(OH)₂ | F₁₅C₁₈H₂₂PO(OH)₂ |
| 20 | | 0.025 (p) |
| | 0.002 (n) | - (n) |
| 70 | 0.6 (p) | |
| | 0.03 (n) | |

### Example 4

### 2,5-Di-n-hoxyl-3,6-bis(5-cyano-thiophon-2-yl)pyrrolo[3,4-c]-pyrrole-1,4-dione (A-3)

The synthesis of 2,5-di-*n*-hexyl-3,6-bis(5-bromo-thiophen-2-yl)pyrrolo[3,4-c]-pyrrole-1,4-dione is done in analogy to Zhou et al., Macromolecules 43 (2010) 821-826.

2,5-Di-*n*-hexyl-3,6-bis(5-bromo-thiophen-2-yl)pyrrolo[3,4-c]-pyrrole-1,4-dione (237 mg, 0.378 mmol) and copper(I)-cyanide (1.50 g, 17.0 mmol) are placed in a 25 mL-flask under argon. Dry DMF (10 mL) is added and the mixture is stirred at 130 °C for 4 h. The solvent is removed under reduced pressure and the residual solid is extracted with chloroform (250 mL). After removing the solvent the crude product is purified by column chromatography (dichloromethane). A violet solid is obtained (52 mg, 27%).

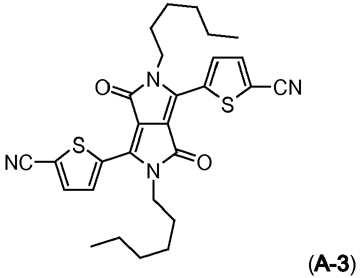

**¹H-NMR** (400 MHz in CD₂Cl₂): δ 8.86 (d, ³*J*= 4.2 Hz, 2H), 7.77 (d, ³*J*= 4.2 Hz, 2H), 4.03 (t, ³*J*= 7.8 Hz, 4H), 1.75-1.65 (m, 4H), 1.46 - 1.27 (m, 12H), 0.90 (t, ³*J*= 7.1 Hz, 6H).
**HRMS** (ESI, pos-mode): calc. for C₂₈H₃₀N₄O₂S₂ 518.1805, found 518.1807.

### Application Example 4

To investigate the behavior of compound **A-3** in an active layer of a field effect transistor, bottom-gate top-contact thin film transistors (TFT) with a 110 nm thick SiO₂/AlOₓ/SAM gate dielectric are fabricated and measured in air. The organic compound is vacuum deposited at 90 °C (Table 4).

**Table 4: Summary of the performance for TFTs with compound A-3 fabricated on SiO₂/AlOₓ/SAM with different SAMs.**

| T_{D} / °C | µ / cm² V⁻¹ s⁻¹ | µ / cm² V⁻¹ s⁻¹ |
|---|---|---|
| SAM | C₁₄H₂₉PO(OH)₂ | F₁₅C₁₈H₂₂PO(OH)₂ |
| 90 | 10⁻⁴ (p) | 0.06 (p) |
| | 0.05 (n) | 0.03 (n) |

### Example 5

### 2,5-Di-n-octyl-3,6-bis(5-cyano-thiophon-2-yl)pyrrolo[3,4-c]-pyrrole-1,4-dione (A-4)

The synthesis of 2,5-di-*n*-octyl-3,6-bis(5-bromo-thiophen-2-yl)pyrrolo[3,4-c]-pyrrole-1,4-dione is done in analogy to Zhou et al., Macromolecules 43 (2010) 821-826.

2,5-Di-*n*-octyl-3,6-bis(5-bromo-thiophen-2-yl)pyrrolo[3,4-c]-pyrrole-1,4-dione (483 mg, 0.708 mmol) and copper(I)-cyanide (1.40 g, 15.6 mmol) in dry DMF (10 mL) are heated under argon at 160 °C for 60 min. Then further 10 mL DMF are added and the mixture is stirred at 165 °C for 140 min. The solvent is removed under reduced pressure and the crude product continuously extracted from the reaction mixture with chloroform. The extract is purified by column chromatography (dichloromethane) and washed with hexane affording 96.5 mg (24%) of a dark violet solid.

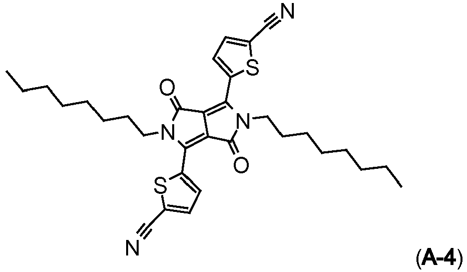

**¹H NMR** (400 MHz, CD₂Cl₂): 8.90 (d, ³*J*= 4.2 Hz, 2H), 7.75 (d, ³*J*= 4.2 Hz, 2H), 4.03 (t, ³*J*= 7.8 Hz), 1.75 - 1.68 (m, 4H), 1.46 - 1.20 (m, 20H), 0.88 (t, ³*J*= 6.9 Hz; 6H).
**HRMS** (ESI, acetonitrile/CHCl₃ 1:1, pos-mode): calc. for C₃₂H₃₉N₄O₂S₂ 575.2509, found: 575.2511.
**Elemental analysis:** calc. for C, 66.87; H, 6.66; N, 9.75; O, 5.57; S, 11.16; found: C, 66.67, H 6.71, N 9.60, S 11.09.

### Example 6

### 2,5-Bis(2-ethylhexyl)-3,6-bis(5'-dicarbonitrile-(2,2'-bithiophenyl-5-yl)-2,5-dihydro-pyrrolo [3,4-c]pyrrole-1,4-dione (A-18)

A degassed 3.1 mL H₂O solution of K₃PO₄ (947 mg, 4.47 mmol) is added to a degassed 27 mL THF solution of 2,5-bis(2-ethylhexyl)-3,6-bis[5-(4,4,5,5-tetramethyl-(1,3,2)dioxoborolan-2-yl)-thiophen-2-yl]-2,5-dihydro-pyrrolo[3,4-*c*]pyrrole-1,4-dione (1.14 g, 1.46 mmol), which can be synthesized in analogy to Example 37 of WO2009/047104,_Pd₂(dba)₃ (39.0 mg, 0.04 mmol), P(*t-*Bu)₃ × HBF₄ (23.0 mg, 0.08 mmol) and 2-bromo-thiophene-5-carbonitrile (1.1 g, 5.85 mmol) under argon atmosphere. After heating to 80 °C for 2 h, the solvent is removed under vacuum. Column chromatography (CH₂Cl₂) afforded 380 mg (0.51 mmol, 35%) of the product **A-18** as a dark blue solid.

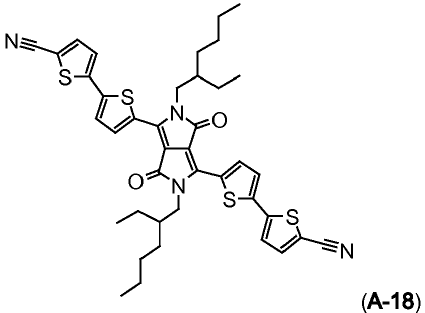

Mp 223-225 °C.
**¹H** NMR (400 MHz, CD₂Cl₂): *δ* 8.90 (d, ³*J*= 4.2 Hz, 2H), 7.60 (d, ³*J*= 4.0 Hz, 2H), 7.46 (d, ³*J*= 4.2 Hz, 2H), 7.31 (m, ³*J*= 4.2 Hz, 2H), 4.02 (m, 4H), 1.87 (m, 2H), 1.32 (m, 16H), 0.89 (m, 12H). **¹³C** NMR (101 MHz, CD₂Cl₂): δ 162.0 (C), 143.5 (C), 140.1 (C), 139.9 (C), 136.8 (CH), 131.1 (C), 127.5 (CH), 125.4 (CH), 114.3 (C), 109.9 (C), 109.7 (C), 46.5 (CH₂), 39.9 (CH), 30.9 (CH₂), 29.1 (CH₂), 24.3 (CH₂), 23.7 (CH₂), 14.4 (CH₃), 10.9 (CH₃).
HRMS (ESI): *m*/*z* calcd. for C₄₀H₄₃N₄O₂S₄ [M+H]⁺ 739.2269, found 739.2261.

**Elemental analysis** (%) calcd. for C₄₀H₄₂N₄O₂S₄: C, 65.01; H, 5.73; N, 7.58; S, 17.35, found: C, 65.03; H, 5.76; N, 7.41; S, 17.39. UV/vis (CH₂Cl₂): *λ*ₘₐₓ/nm (*ε*ₘₐₓ/M⁻¹ cm⁻¹) 586 (46600).

### Example 7

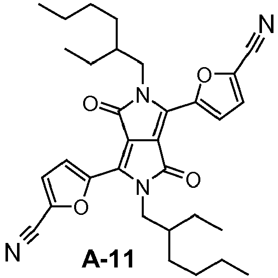

The compound of formula **A-11** is synthesized in analogy to example 1, starting from **[1254943-39-9]** (Synthesis described in JACS 2010 132(44) 15547). Mass spectrum (APCI, negative mode): 543.3.

### Example 8

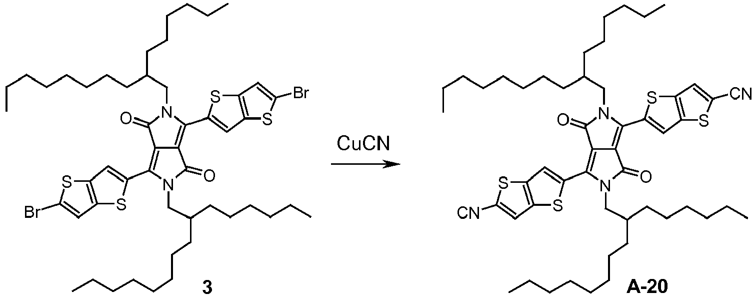

The compound of formula **A-20** is synthesized in analogy to example 1, starting from compound **3** (the synthesis of compound **3** is done in analogy to example 1 of WO2010108873). Mass spectrum (APCI, positive mode): 911.13.

## Claims

1. A compound of the formula 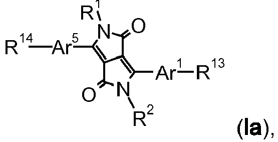 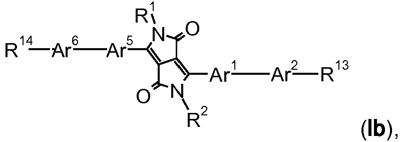 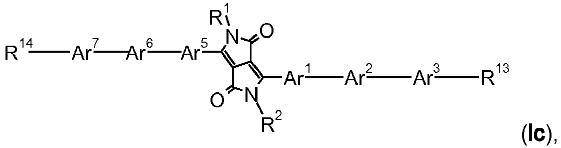 or 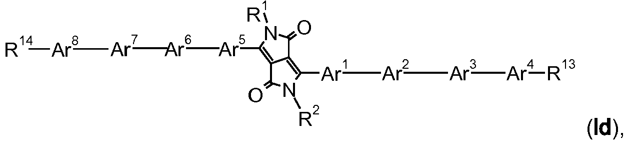 wherein
R¹ and R² are selected from a C₁-C₃₈alkyl group, a C₁-C₃₈alkyl group which is substituted by one or more halogen atoms, especially a F containing C₂-C₃₆alkyl group, very especially CH₂CₙF₂ₙ₊₁ (n = 1 to 10); a C₇-C₂₅arylalkyl group, which can be substituted one to five times with C₁-C₈alkyl, C₁-C₈alkoxy and/or F, especially 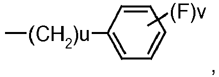 or 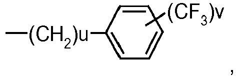 u is 1, or 2, v is 1 to 5; and pentafluorophenyl;
Ar¹ and Ar⁵ are independently of each other 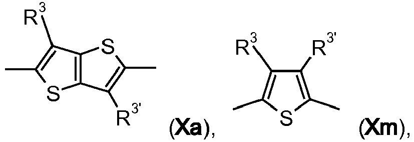 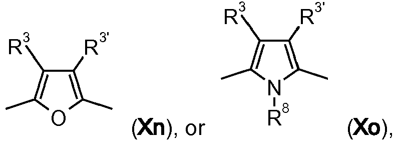 wherein R³ and R^{3'} are independently of each other hydrogen, halogen, CF₃, CN, C₁-C₂₅alkyl or C₁-C₂₅alkoxy, and R⁸ is hydrogen or C₁-C25alkyl;
Ar², Ar³, Ar⁴, Ar⁶, Ar⁷ and Ar⁸ are independently of each other 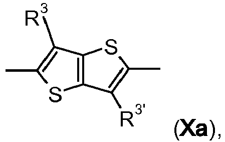 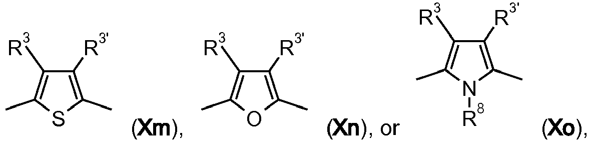 wherein R³ and R^{3'} are independently of each other hydrogen, halogen, CF₃, CN, C₁-C₂₅alkyl or C₁-C₂₅alkoxy, and R⁸ is hydrogen or C₁-C₂₅alkyl; and
R¹³ and R¹⁴ are CN, or

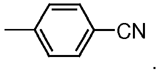

2. A compound according to claim 1, wherein R¹³ and R¹⁴ represent CN.

3. A compound according to claim 1, or 2, wherein R³ and R^{3'} are independently of each other hydrogen, or C₁-C₂₅alkyl.

4. A compound according to any of claims 1 to 3, wherein Ar¹ and Ar⁵ are independently of each other

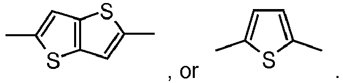

5. A compound according to any of claims 1 to 4, wherein Ar², Ar³, Ar⁴, Ar⁶, Ar⁷ and Ar⁸ are independently of each other

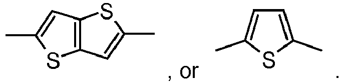

6. A compound according to any of claims 1 to 5, wherein R¹ and R² are a C₁-C₃alkyl group.

7. A compound according to claim 1, which is a compound of the formula 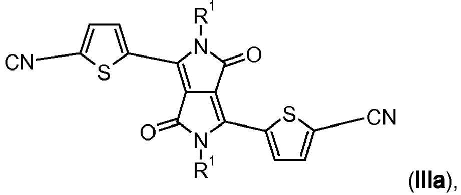 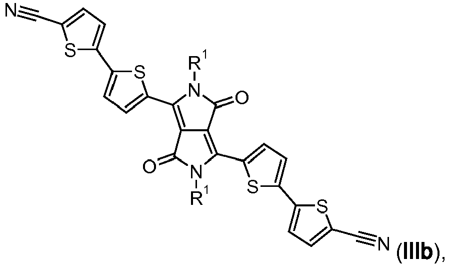 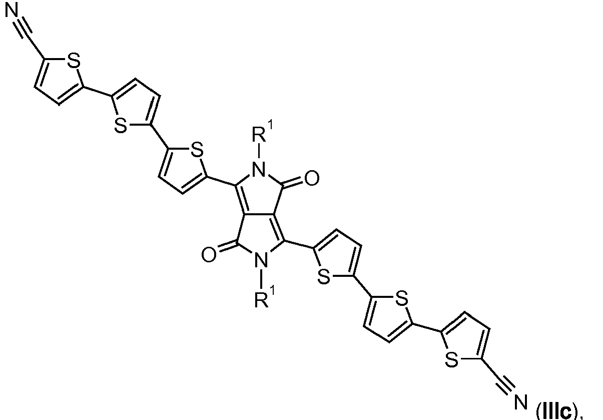 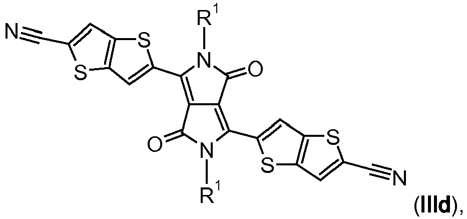 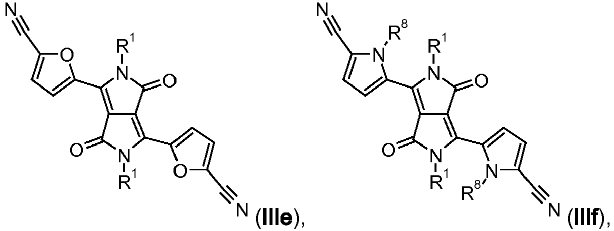 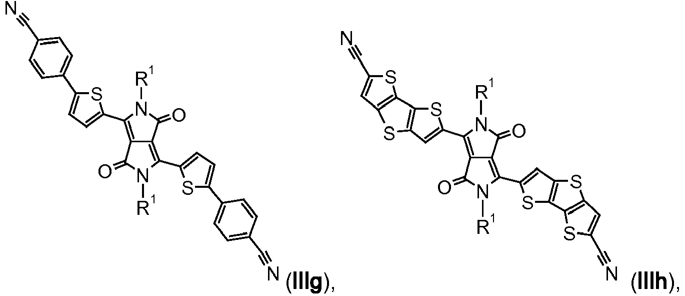 or 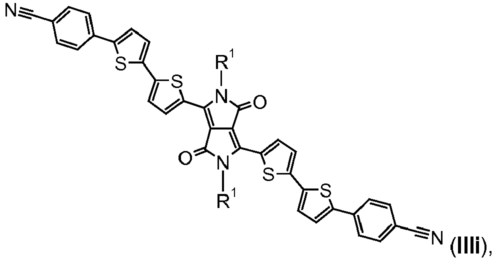 wherein R¹ is a C₁-C₃₈alkyl group, especially C₂-C₁₂alkyl group, a F containing C₂-C₃₆alkyl group, especially CH₂CₙF₂ₙ₊₁ (n = 1 to 10), or 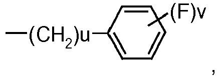 or 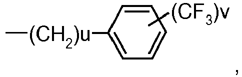 u is 1, or 2, v is 1 to 5, and R⁸ is hydrogen or C₁-C₂₅alkyl.

8. A compound of the formula I according to claim 1, selected from the compounds having the following formulae

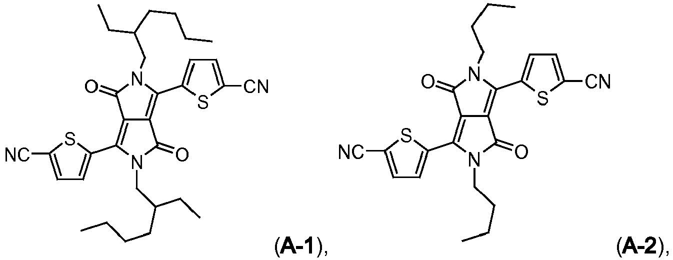

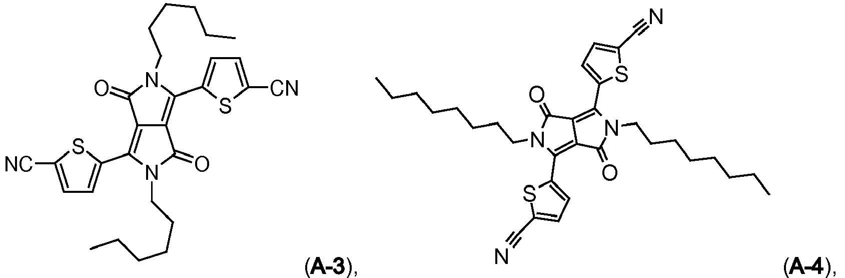

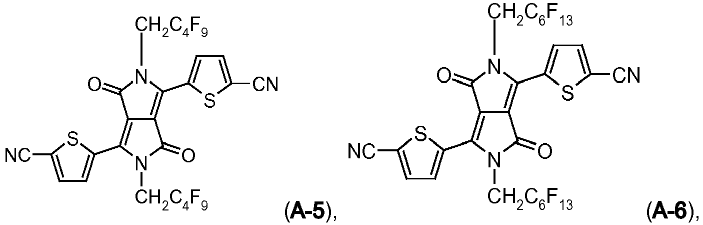

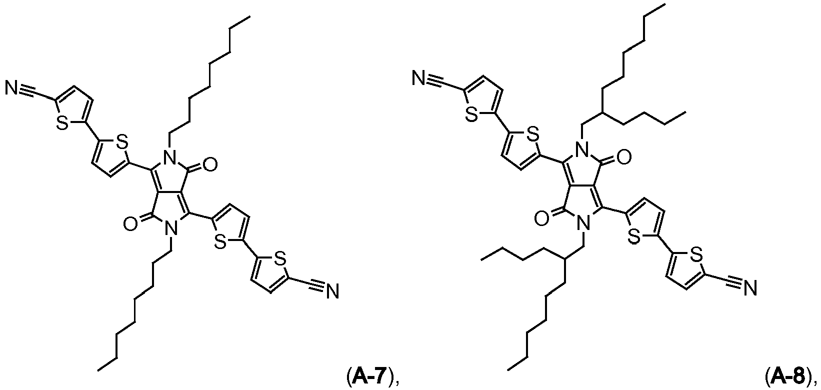

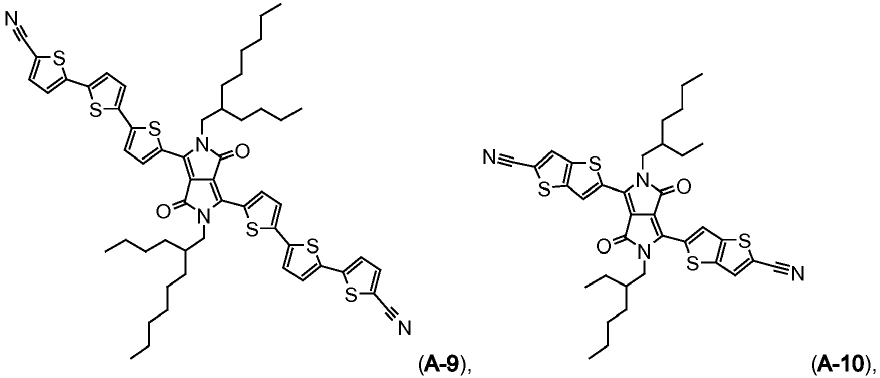

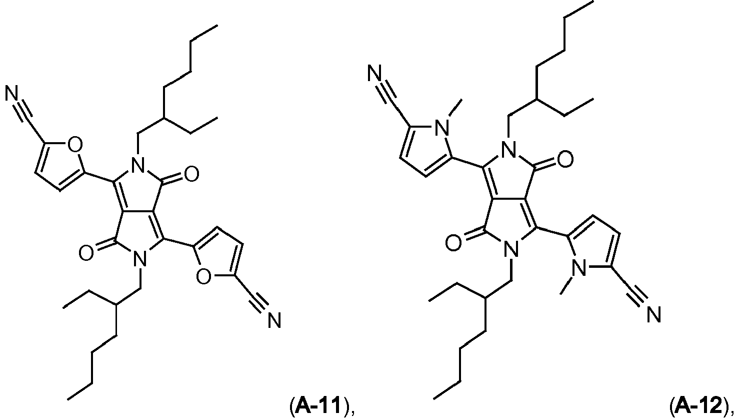

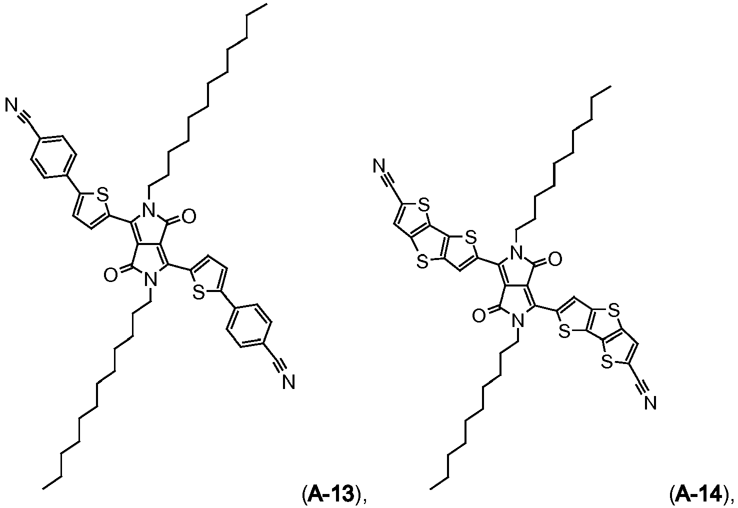

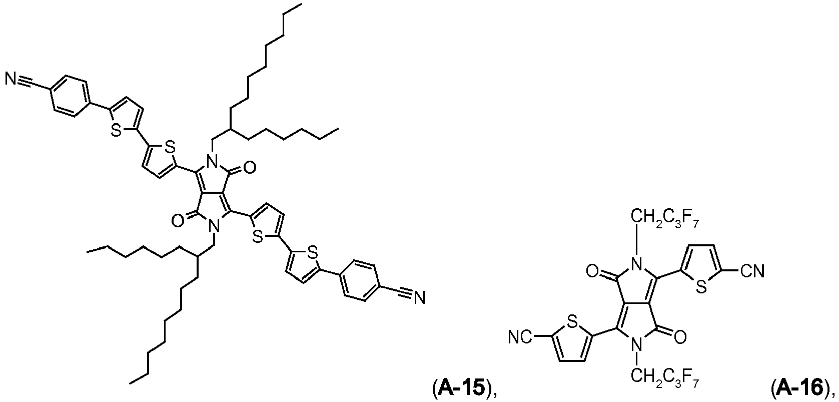

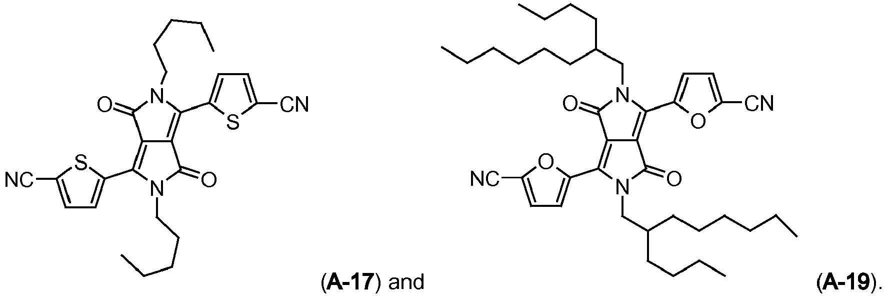

9. An organic semiconductor material, layer or component, comprising a compound according to any of claims 1 to 8.

10. A semiconductor device comprising as a semiconducting effective means a compound according to any of claims 1 to 8, or an organic semiconductor material, layer or component according to claim 9.

11. A semiconductor device according to claim 10 in the form of a diode, a photodiode, a sensor, an organic field effect transistor, a transistor for flexible displays, a RFID, or a (heterojunction) solar cell.

12. Use of a compound according to any one of claims 1 to 8 as a semiconductor.

13. A process for the preparation of an organic semiconductor device, wherein the organic semiconductor layer is formed by applying a solution and/or dispersion of a compound according to any of claims 1 to 8 in an organic solvent to a suitable substrate and removing the solvent; or the organic semiconductor layer is formed by vacuum vapor deposition of a compound according to any of claims 1 to 8.

14. A process for the preparation of a compound of formula 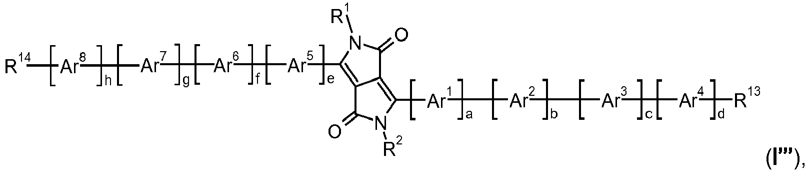 which process comprises reacting a compound of formula 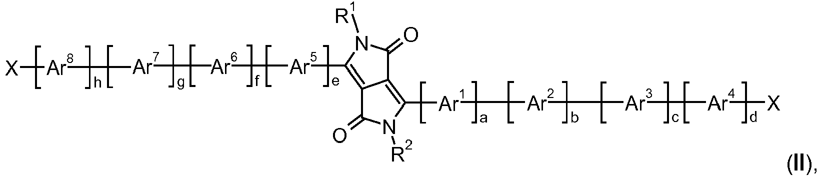 with copper(I)cyanide; or a process for the preparation of a compound of formula 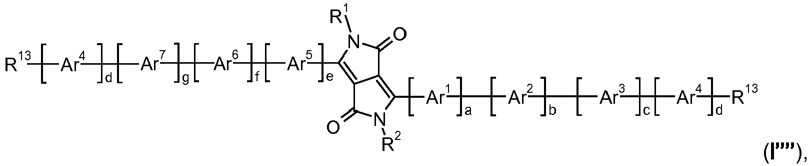 which process comprises reacting a compound of formula 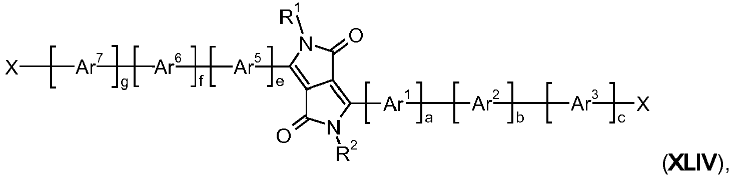
with an equimolar amount of a diboronic acid or diboronate corresponding to formula X¹¹-A²-R¹³ in a solvent and in the presence of a catalyst, or
with an equimolar amount of an organo tin compound corresponding to formula X^{11'}-A²-R¹³
or a process for the preparation of a compound of formula 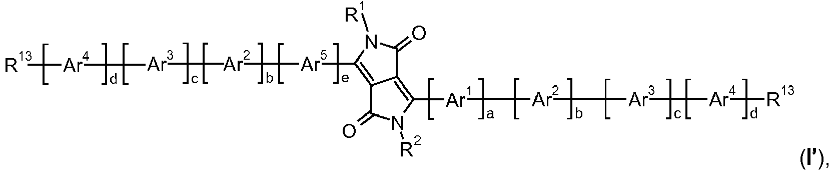
which process comprises reacting a compound of formula 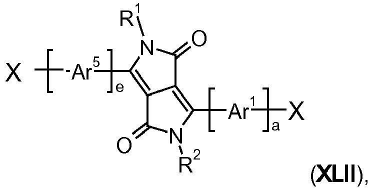
with an equimolar amount of a diboronic acid or diboronate corresponding to formula X¹¹-A-R¹³ in a solvent and in the presence of a catalyst; or
with an equimolar amount of an organo tin compound corresponding to formula X^{11'}-A-R¹³; or a process for the preparation of a compound of formula 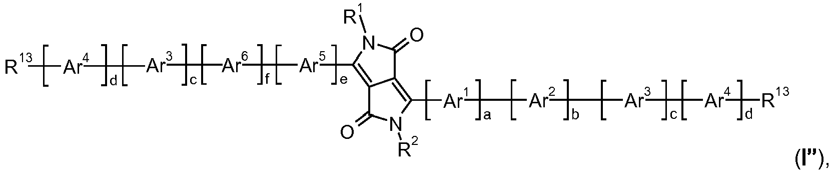
which process comprises reacting a compound of formula 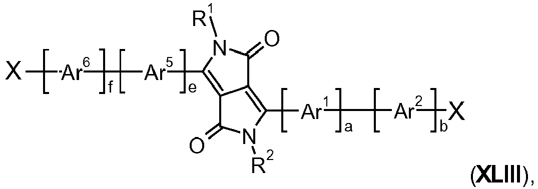
with an equimolar amount of a diboronic acid or diboronate corresponding to formula X¹¹-A¹-R¹³ in a solvent and in the presence of a catalyst; or
with an equimolar amount of an organo tin compound corresponding to formula X^{11'}-A¹-R¹³; wherein X¹¹ is independently in each occurrence -B(OH)₂, -B(OY¹)₂, 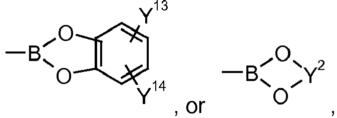 wherein Y¹ is independently in each occurrence a C₁-C₁₈alkylgroup and Y² is independently in each occurrence a C₂-C₁₀alkylene group, such as -CY³Y⁴-CY⁵Y⁶-, or -CY⁷Y⁸-CY⁹Y¹⁰-CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup,
especially -C(CH₃)₂C(CH₃)₂-, -C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup, in a solvent and in the presence of a catalyst;
X^{11'} is independently in each occurrence -SnR²⁰⁷R²⁰⁸R²⁰⁹, wherein R²⁰⁷, R²⁰⁸ and R²⁰⁹ are identical or different and are H or C₁-C₆alkyl, or two of the groups R²⁰⁷, R²⁰⁸ and R²⁰⁹ form a ring and these groups are optionally branched,
R¹³ and R¹⁴ are CN,
A is a group of formula 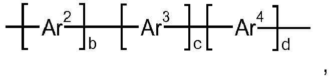
A¹ is a group of formula 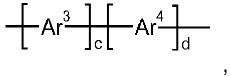
A² is a group of formula 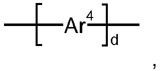
X is Cl, Br, or I;
a is 1, and e is 1,
b, c, d, f, g and h independently of each other represent 0, or 1,
R¹, R², Ar¹, Ar², Ar³, Ar⁴, Ar⁵, Ar⁶, Ar⁷ and Ar⁸ are as defined in claim 1.

## Patentansprüche

1. Verbindung der Formel 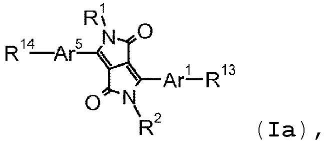 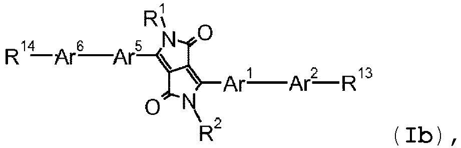 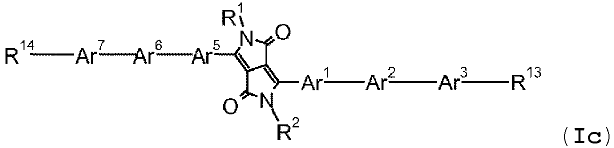 oder 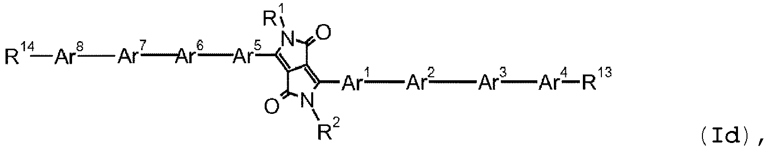 wobei
R¹ und R² aus einer C₁-C₃₈-Alkylgruppe, einer C₁-C₃₈-Alkylgruppe, die durch ein oder mehrere Halogenatome substituiert ist, insbesondere einer F enthaltenden C₂-C₃₆-Alkylgruppe, ganz besonders CH₂CₙF₂ₙ₊₁ (n = 1 bis 10), einer C₇-C₂₅-Arylalkyl-gruppe, die ein- bis fünffach durch C₁-C₈-Alkyl, C₁-C₈-Alkoxy und/oder F substituiert sein kann, insbesondere 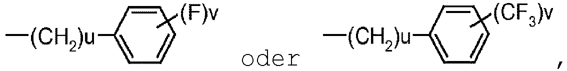 u für 1 oder 2 steht, v für 1 bis 5 steht, und Pentafluorphenyl ausgewählt sind;
Ar¹ und Ar⁵ unabhängig voneinander für 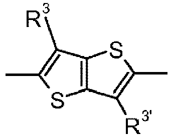 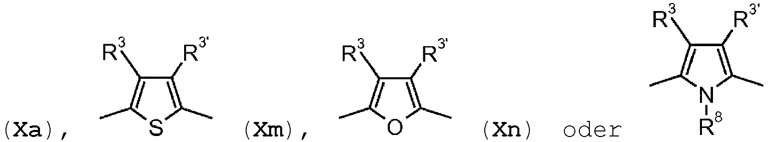 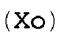 stehen, wobei R³ und R^{3'} unabhängig voneinander für Wasserstoff, Halogen, CF₃, CN, C₁-C₂₅-Alkyl oder C₁-C₂₅-Alkoxy stehen und R⁸ für Wasserstoff oder C₁-C₂₅-Alkyl steht;
Ar², Ar³, Ar⁴, Ar⁶, Ar⁷ und Ar⁸ unabhängig voneinander für 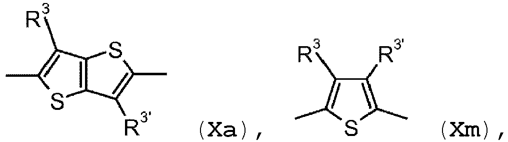 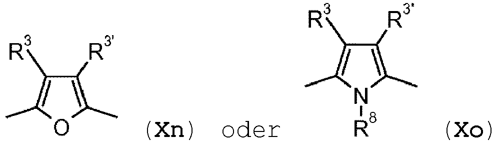 stehen, wobei R³ und R^{3'} unabhängig voneinander für Wasserstoff, Halogen, CF₃, CN, C₁-C₂₅-Alkyl oder C₁-C₂₅-Alkoxy stehen und R⁸ für Wasserstoff oder C₁-C₂₅-Alkyl steht; und
R¹³ und R¹⁴ für CN oder 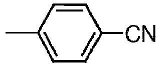 stehen.

2. Verbindung nach Anspruch 1, wobei R¹³ und R¹⁴ für CN stehen.

3. Verbindung nach Anspruch 1 oder 2, wobei R³ und R^{3'} unabhängig voneinander für Wasserstoff oder C₁-C₂₅-Alkyl stehen.

4. Verbindung nach einem der Ansprüche 1 bis 3, wobei Ar¹ und Ar⁵ unabhängig voneinander für 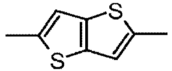 oder 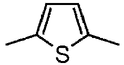 stehen.

5. Verbindung nach einem der Ansprüche 1 bis 4, wobei Ar², Ar³, Ar⁴, Ar⁶, Ar⁷ und Ar⁸ unabhängig voneinander für 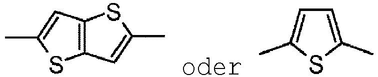 stehen.

6. Verbindung nach einem der Ansprüche 1 bis 5, wobei R¹ und R² für eine C₁-C₃₈-Alkylgruppe stehen.

7. Verbindung nach Anspruch 1, bei der es sich um eine Verbindung der Formel 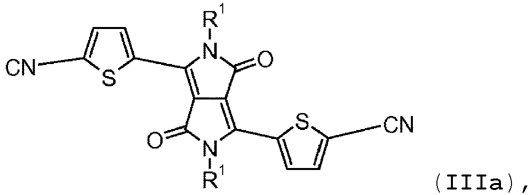 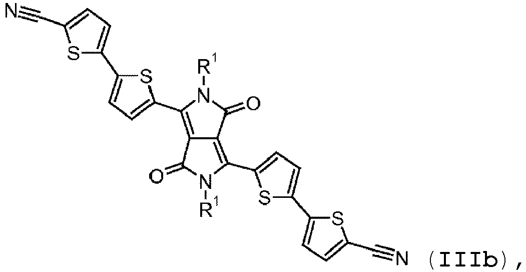 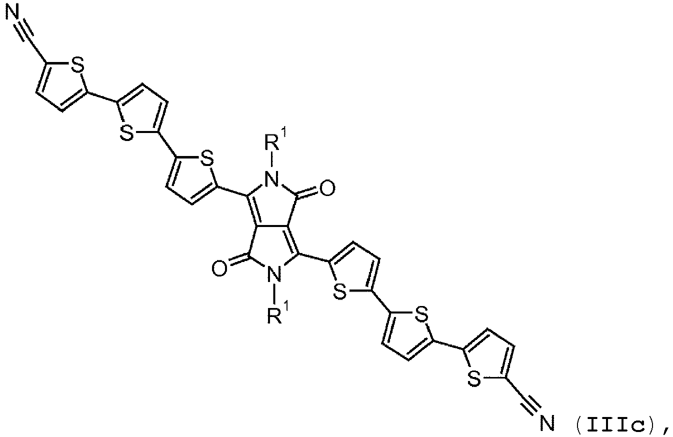 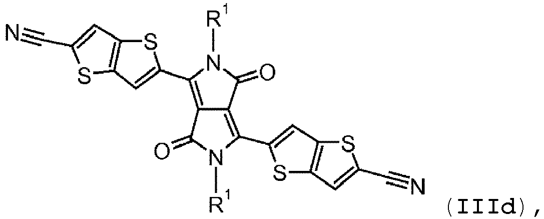 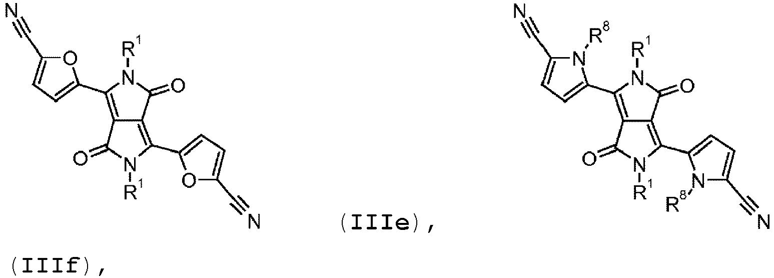 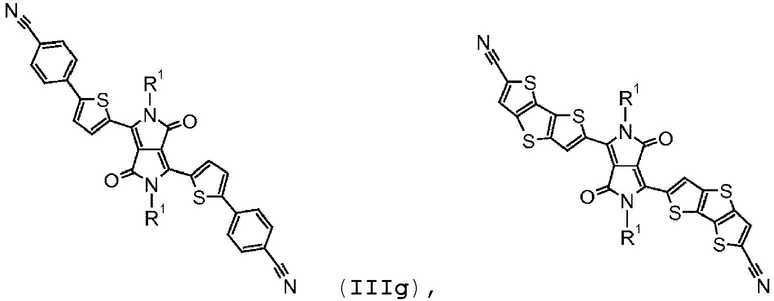 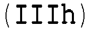 oder 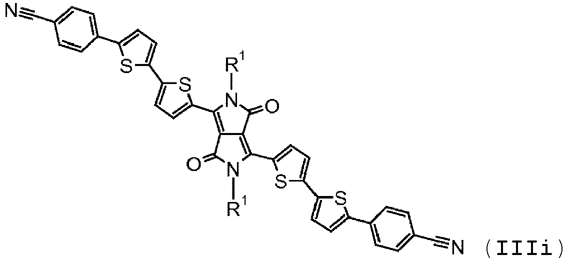 handelt, wobei R¹ für eine C₁-C₃₈-Alkylgruppe, insbesondere C₂-C₁₂-Alkylgruppe, eine F enthaltende C₂-C₃₆-Alkylgruppe, insbesondere CH₂CₙF₂ₙ₊₁ (n = 1 bis 10), oder 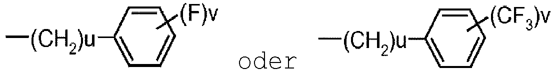 steht, u für 1 oder 2 steht, v für 1 bis 5 steht und
R⁸ für Wasserstoff oder C₁-C₂₅-Alkyl steht.

8. Verbindung der Formel I nach Anspruch 1, ausgewählt aus den Verbindungen mit den folgenden Formeln: 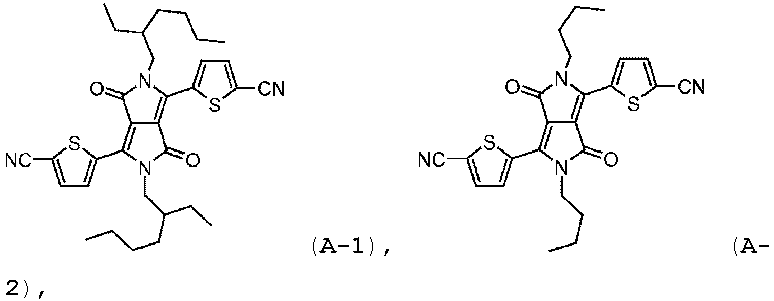 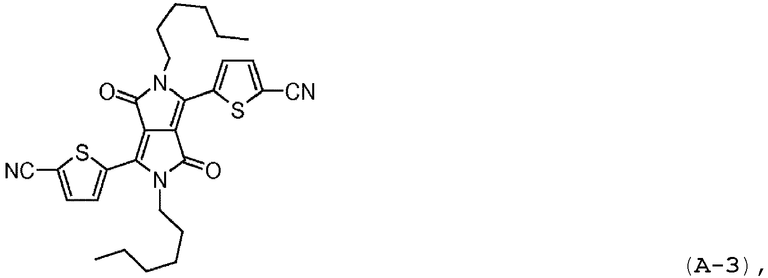 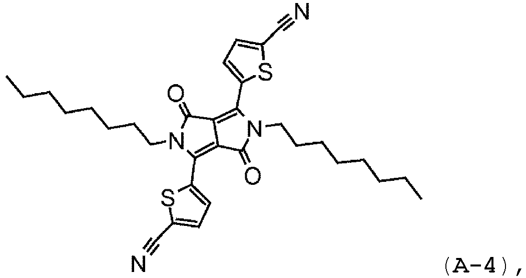 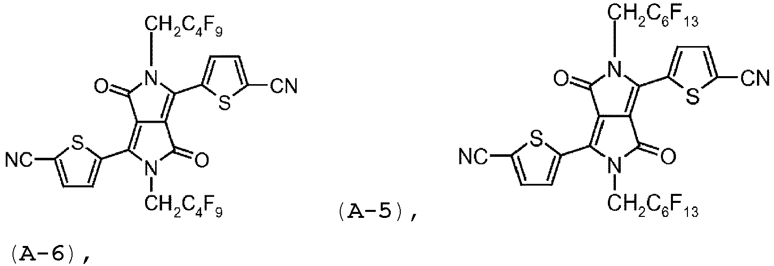 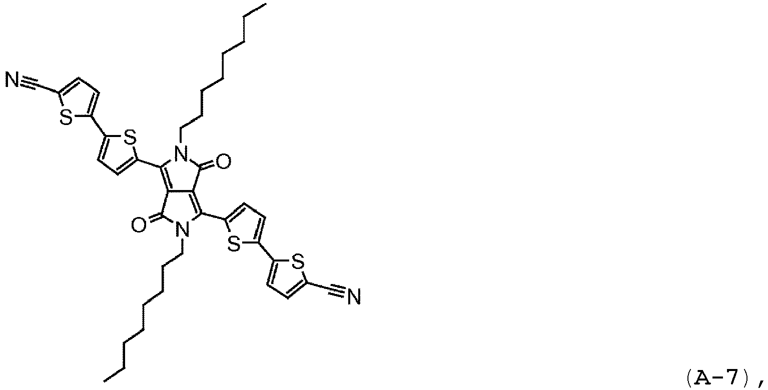 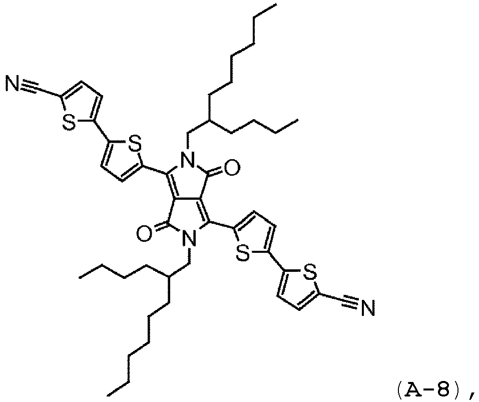 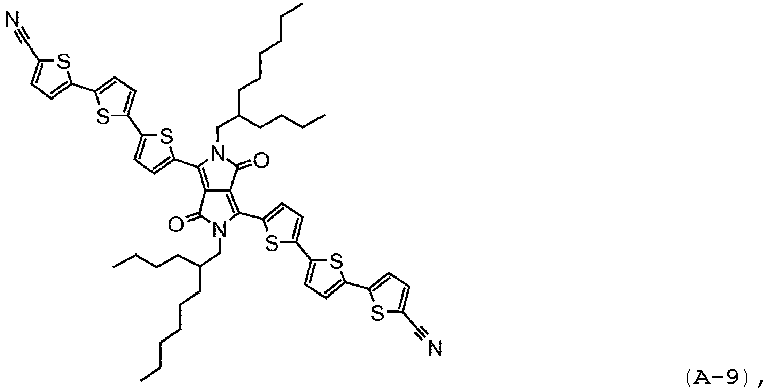 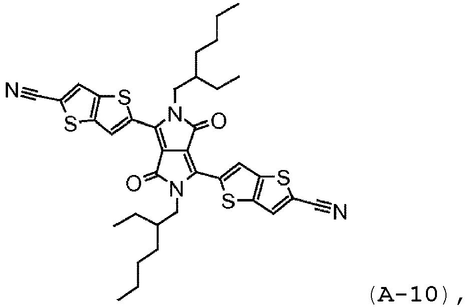 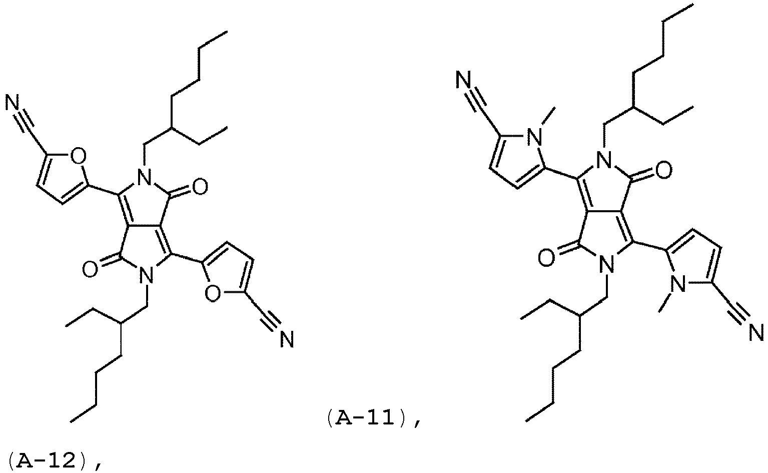 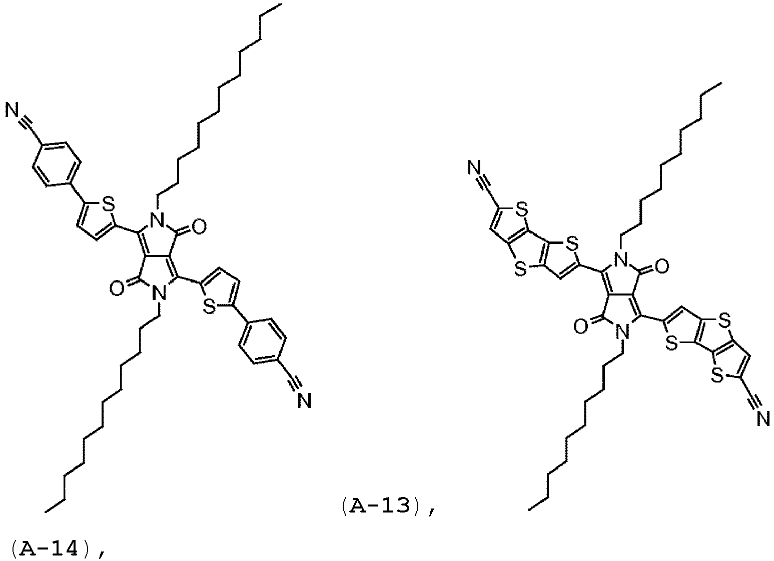 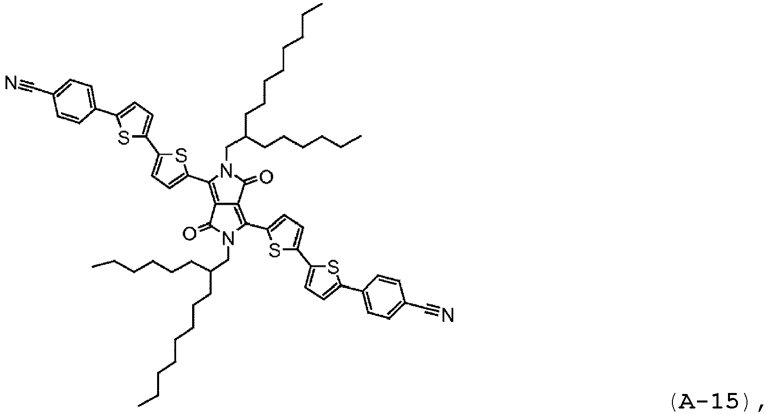 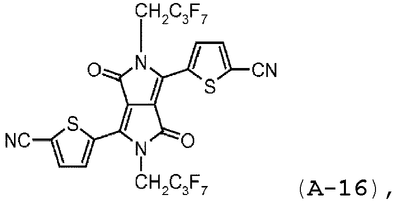 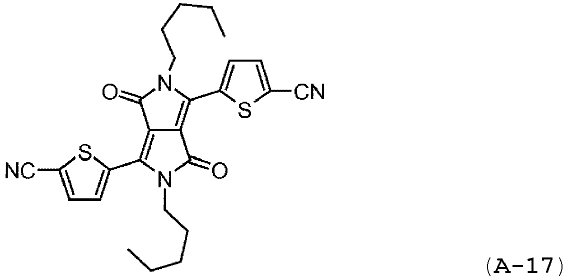 und

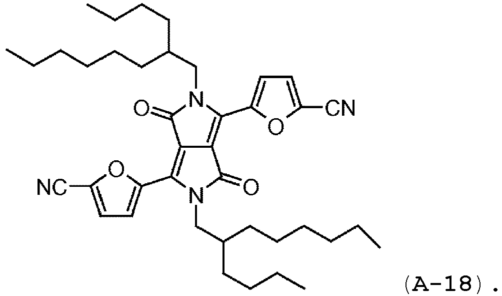

9. Organisches Halbleitermaterial, organische Halbleiterschicht oder organisches Halbleiterbauelement, umfassend eine Verbindung nach einem der Ansprüche 1 bis 8.

10. Halbleitervorrichtung, umfassend als halbleitungswirksames Mittel eine Verbindung nach einem der Ansprüche 1 bis 8 oder ein organisches Halbleitermaterial, eine organische Halbleiterschicht oder ein organisches Halbleiterbauelement nach Anspruch 9.

11. Halbleitervorrichtung nach Anspruch 10 in Form einer Diode, einer Photodiode, eines Sensors, eines organischen Feldeffekttransistors, eines Transistors für flexible Displays, eines RFID oder einer Solarzelle (mit Heteroübergang).

12. Verwendung einer Verbindung nach einem der Ansprüche 1 bis 8 als Halbleiter.

13. Verfahren zur Herstellung einer organischen Halbleitervorrichtung, bei dem die organische Halbleiterschicht durch Aufbringen einer Lösung und/oder Dispersion einer Verbindung nach einem der Ansprüche 1 bis 8 in einem organischen Lösungsmittel auf ein geeignetes Substrat und Entfernen des Lösungsmittels gebildet wird; oder die organische Halbleiterschicht durch Vakuumdampfabscheidung einer Verbindung nach einem der Ansprüche 1 bis 8 gebildet wird.

14. Verfahren zur Herstellung einer Verbindung der Formel 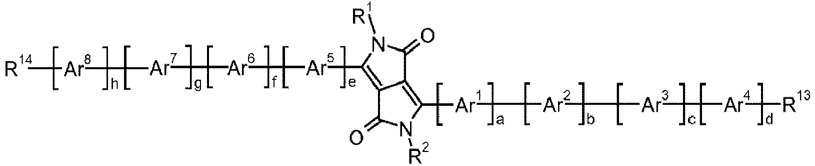 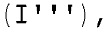 bei dem man eine Verbindung der Formel 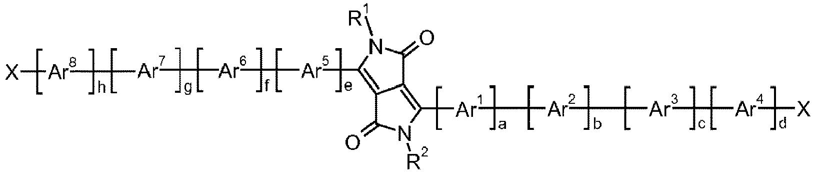 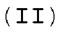 mit Kupfer(I)-cyanid umsetzt; oder Verfahren zur Herstellung einer Verbindung der Formel 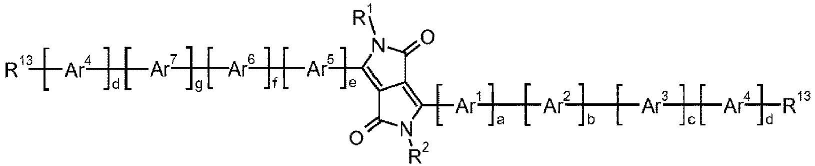 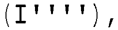 bei dem man eine Verbindung der Formel 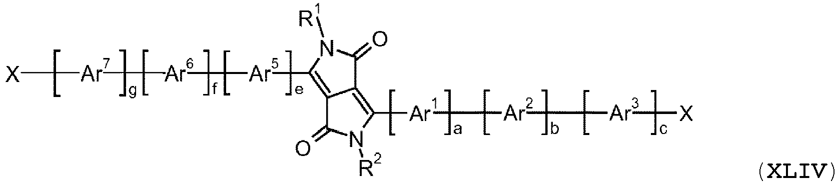
in einem Lösungsmittel und in Gegenwart eines Katalysators mit einer äquimolaren Menge einer Diboronsäure oder eines Diboronats der Formel X¹¹-A²-R¹³ oder
mit einer äquimolaren Menge einer Organozinnverbindung der Formel X^{11'}-A²-R¹³ umsetzt;
oder Verfahren zur Herstellung einer Verbindung der Formel 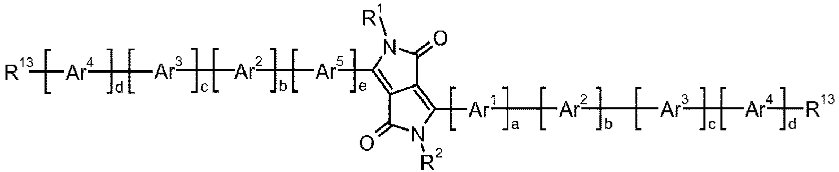 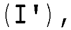 bei dem man eine Verbindung der Formel 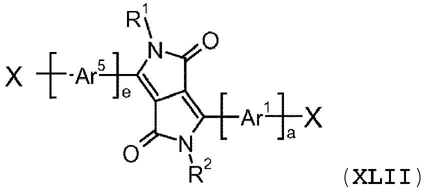
in einem Lösungsmittel und in Gegenwart eines Katalysators mit einer äquimolaren Menge einer Diboronsäure oder eines Diboronats der Formel X¹¹-A-R¹³ oder
mit einer äquimolaren Menge einer Organozinnverbindung der Formel X^{11'}-A-R¹³ umsetzt; oder Verfahren zur Herstellung einer Verbindung der Formel 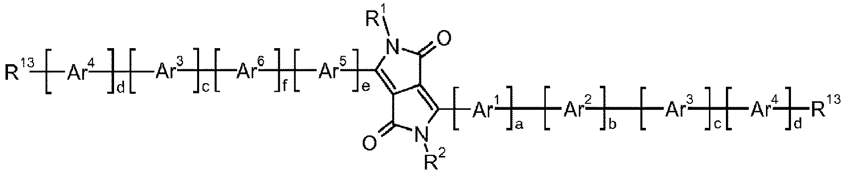 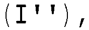 bei dem man eine Verbindung der Formel 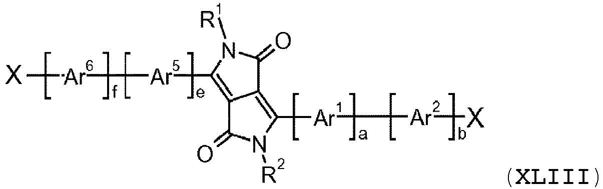
in einem Lösungsmittel und in Gegenwart eines Katalysators mit einer äquimolaren Menge einer Diboronsäure oder eines Diboronats der Formel X¹¹-A¹-R¹³ oder
in einem Lösungsmittel und in Gegenwart eines Katalysators mit einer äquimolaren Menge einer Organozinnverbindung der Formel X^{11'}A¹-R¹³ umsetzt; wobei X¹¹ unabhängig bei jedem Auftreten für -B(OH)₂, -B(OY¹)₂, 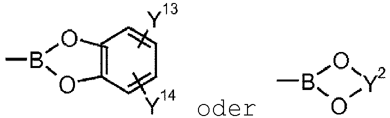 steht, wobei Y¹ unabhängig bei jedem Auftreten für eine C₁-C₁₈-Alkylgruppe steht und Y² unabhängig bei jedem Auftreten für eine C₂-C₁₀-Alkylengruppe, wie -CY³Y⁴-CY⁵Y⁶- oder -CY⁷Y⁸-CY⁹Y¹⁰-CY¹¹Y¹²-, steht, wobei Y³, Y⁴, Y⁵, Y⁶, Y⁷, Y⁸, Y⁹, Y¹⁰, Y¹¹ und Y¹² unabhängig voneinander für Wasserstoff oder eine C₁-C₁₈-Alkylgruppe, insbesondere -C(CH₃)₂C(CH₃)₂-, -C(CH₃)₂CH₂C(CH₃)₂ oder -CH₂C(CH₃)₂CH₂- stehen und Y¹³ und Y¹⁴ unabhängig voneinander für Wasserstoff oder eine C₁-C₁₈-Alkylgruppe stehen;
X^{11'} unabhängig bei jedem Auftreten für -SnR²⁰⁷R²⁰⁸R²⁰⁹ steht, wobei R²⁰⁷, R²⁰⁸ und R²⁰⁹ gleich oder verschieden sind und für H oder C₁-C₆-Alkyl stehen oder zwei der Gruppen R²⁰⁷, R²⁰⁸ und R²⁰⁹ einen Ring bilden und diese Gruppen gegebenenfalls verzweigt sind,
R¹³ und R¹⁴ für CN stehen,
A für eine Gruppe der Formel 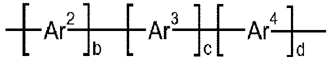 steht,
A¹ für eine Gruppe der Formel 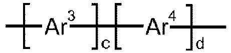 steht,
A² für eine Gruppe der Formel 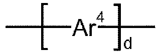 steht,
X für Cl, Br oder I steht;
a für 1 steht und e für 1 steht,
b, c, d, f, g und h unabhängig voneinander für 0 oder 1 stehen,
R¹, R², Ar¹, Ar², Ar³, Ar⁴, Ar⁵, Ar⁶, Ar⁷ und Ar⁸ wie in Anspruch 1 definiert sind.

## Revendications

1. Composé de la formule 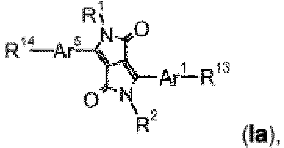 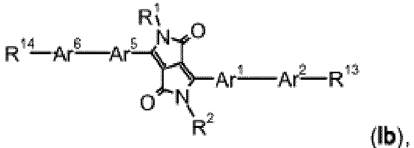 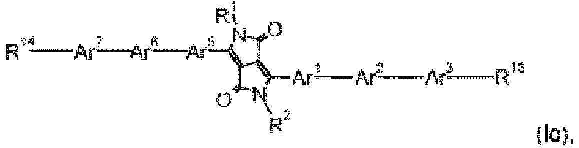 ou 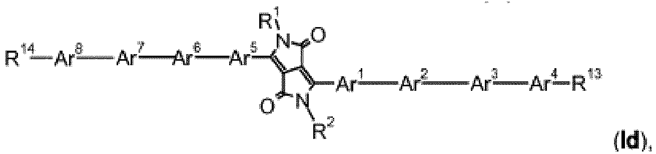
R¹ et R² étant choisis parmi un groupe C₁₋₃₈-alkyle, un groupe C₁₋₃₈-alkyle qui est substitué par un ou plusieurs atomes d'halogène, particulièrement un groupe C₂₋₃₆-alkyle contenant F, très particulièrement CH₂CₙF₂ₙ₊₁ (n = 1 à 10) ; un groupe C₇₋₂₅-arylalkyle, qui peut être substitué une à cinq fois par C₁₋₈-alkyle, C₁₋₈-alcoxyle et/ou F, particulièrement 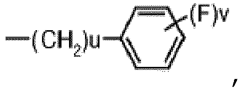 ou 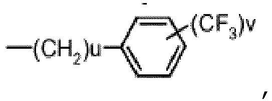 u étant 1, ou 2, v étant 1 à 5 ; et pentafluorophényle ;
Ar¹ et Ar⁵ étant indépendamment l'un de l'autre 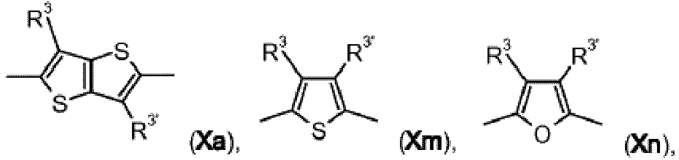 ou 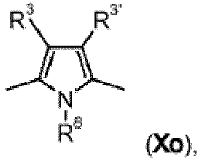 R³ et R^{3'} étant indépendamment l'un de l'autre hydrogène, halogène, CF₃, CN, C₁₋₂₅-alkyle ou C₁₋₂₅-alcoxyle, et R⁸ étant hydrogène ou C₁₋₂₅-alkyle ;
Ar², Ar³, Ar⁴, Ar⁶, Ar⁷ et Ar⁸ étant indépendamment les uns des autres 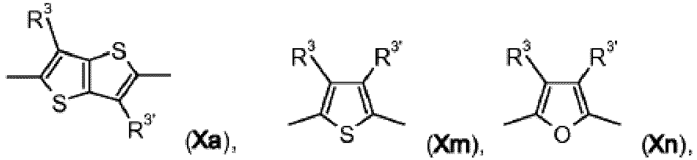 ou 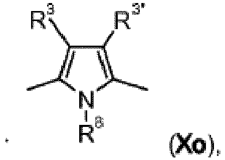 R³ et R^{3'} étant indépendamment l'un de l'autre hydrogène, halogène, CF₃, CN, C₁₋₂₅-alkyle ou C₁₋₂₅-alcoxyle, et
R⁸ étant hydrogène ou C₁₋₂₅-alkyle ; et
R¹³ et R¹⁴ étant CN, ou

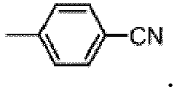

2. Composé selon la revendication 1, R¹³ et R¹⁴ représentant CN.

3. Composé selon la revendication 1 ou 2, R³ et R^{3'} étant indépendamment l'un de l'autre hydrogène, ou C₁₋₂₅-alkyle.

4. Composé selon l'une quelconque des revendications 1 à 3, Ar¹ et Ar⁵ étant indépendamment l'un de l'autre

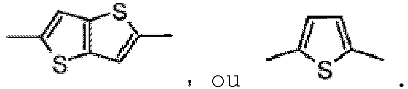

5. Composé selon l'une quelconque des revendications 1 à 4, Ar², Ar³, Ar⁴, Ar⁶, Ar⁷ et Ar⁸ étant indépendamment les uns des autres 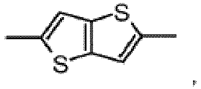 ou

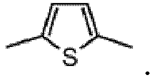

6. Composé selon l'une quelconque des revendications 1 à 5, R¹ et R² étant un groupe C₁₋₃₈-alkyle.

7. Composé selon la revendication 1, qui est un composé de la formule 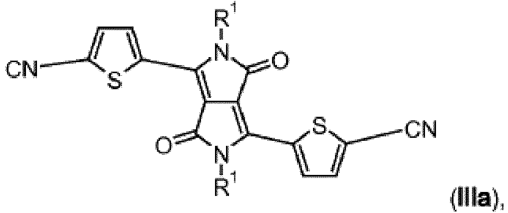 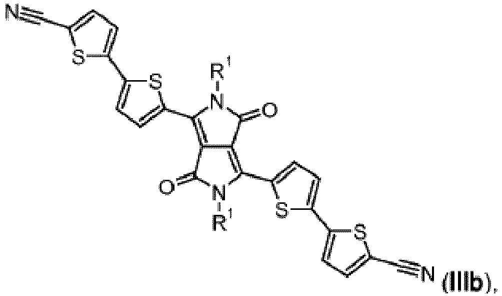 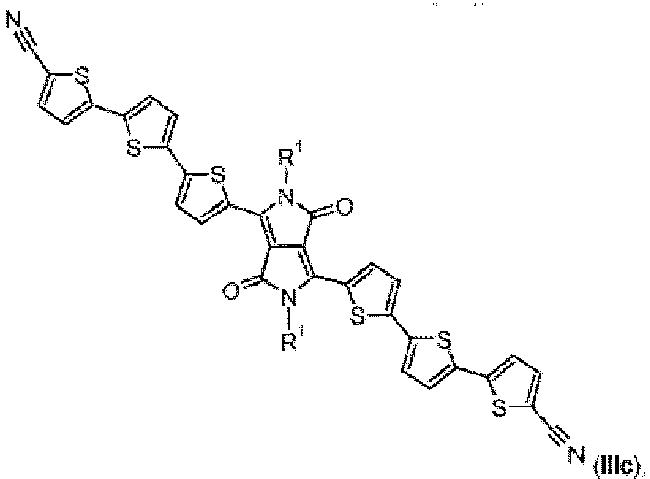 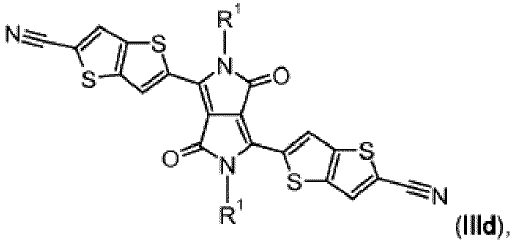 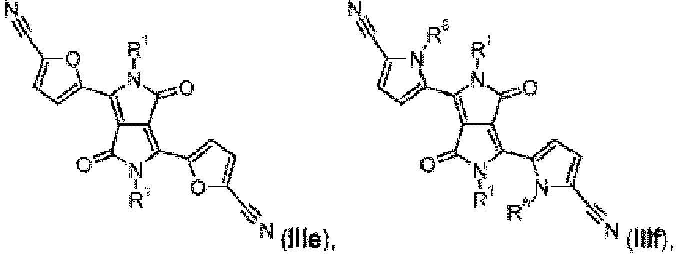 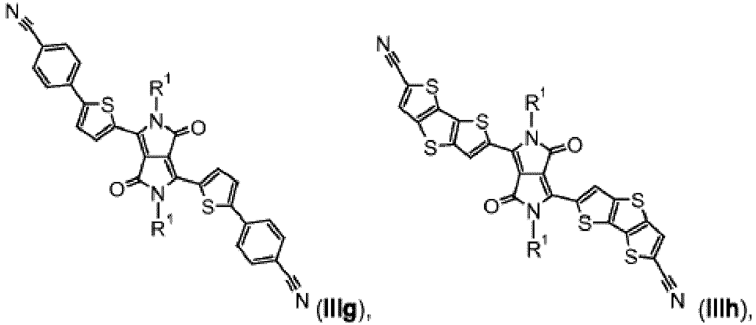 ou 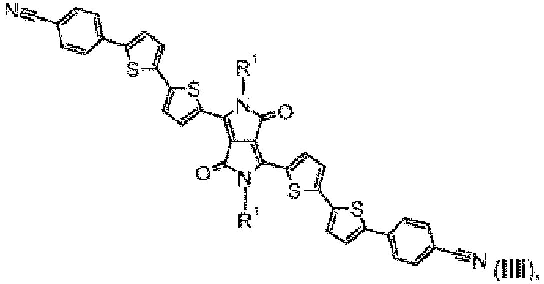
R¹ étant un groupe C₁₋₃₈-alkyle, particulièrement un groupe C₂₋₁₂-alkyle, un groupe C₂₋₃₆-alkyle contenant F, particulièrement CH₂CₙF₂ₙ₊₁ (n = 1 à 10), ou 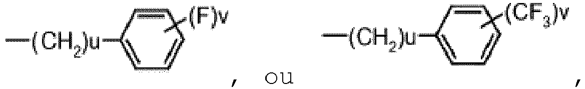 u étant 1, ou 2, v étant 1 à 5, et
R⁸ étant hydrogène ou C₁₋₂₅-alkyle.

8. Composé de la formule I selon la revendication 1, choisi parmi les composés possédant les formules suivantes 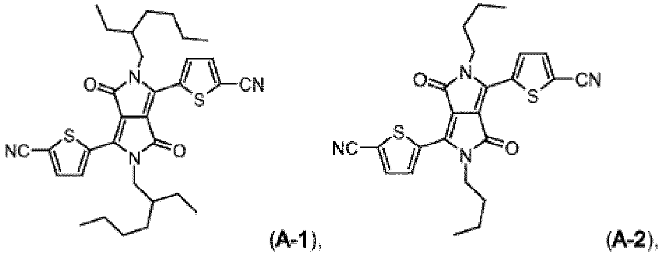 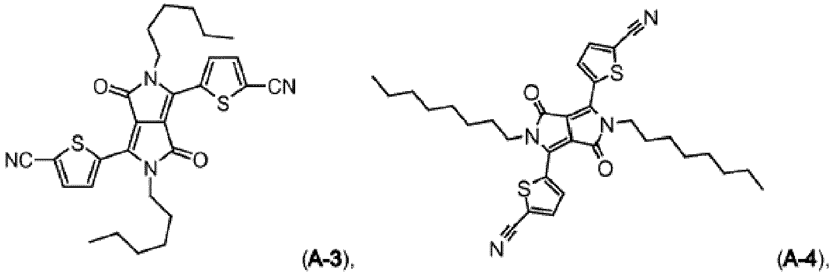 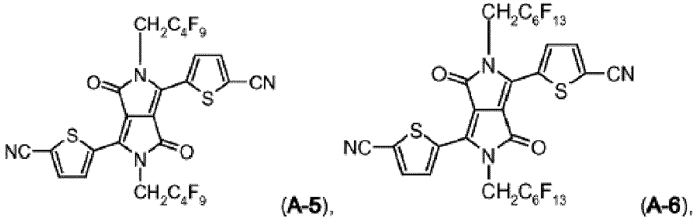 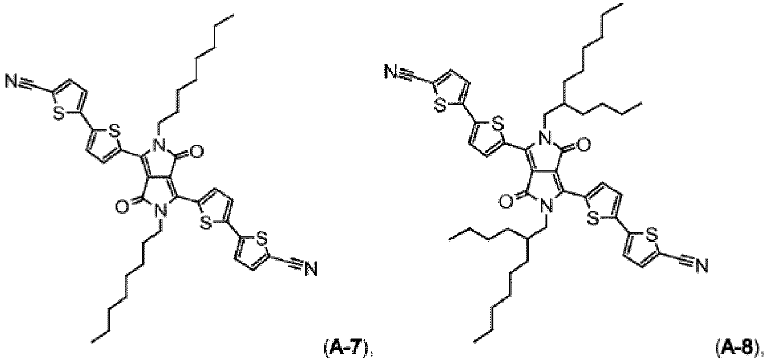 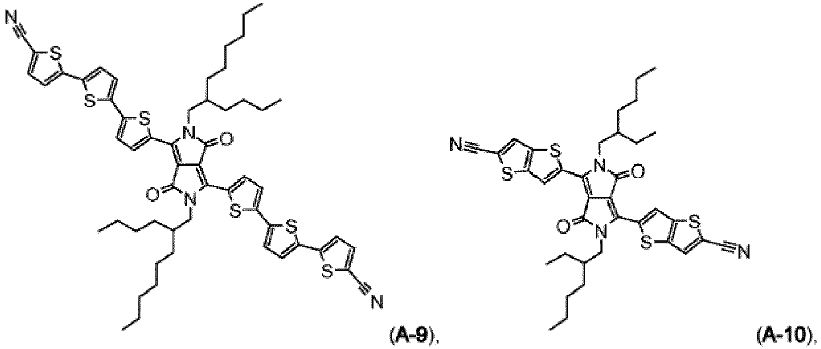 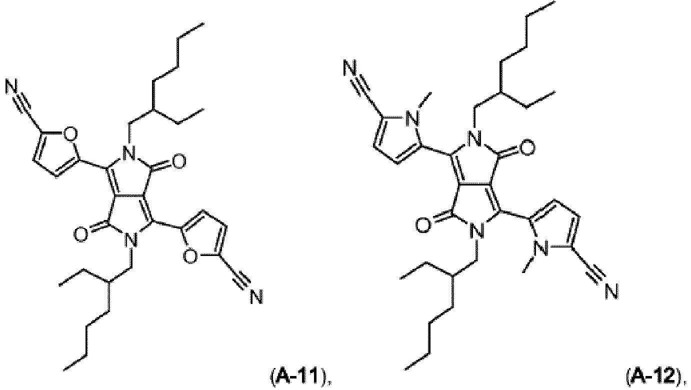 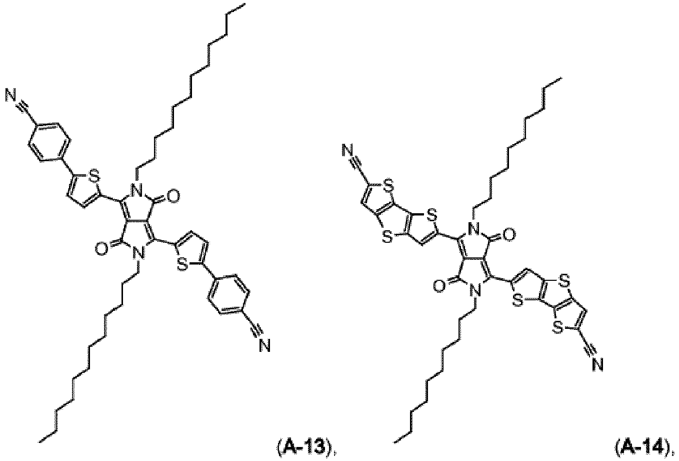 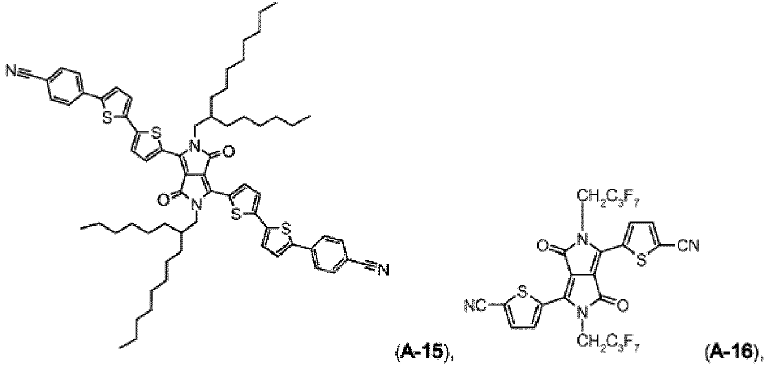 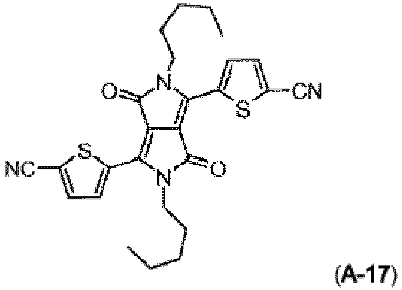 et

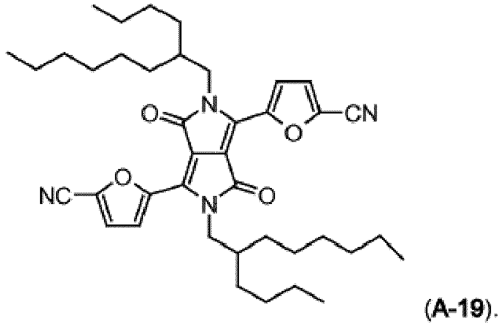

9. Matériau, couche ou composant semi-conducteur/semi-conductive organique, comprenant un composé selon l'une quelconque des revendications 1 à 8.

10. Dispositif semi-conducteur comprenant en tant que moyen efficace semi-conducteur un composé selon l'une quelconque des revendications 1 à 8, ou un matériau, une couche ou un composant semi-conducteur/semi conductrice organique selon la revendication 9.

11. Dispositif semi-conducteur selon la revendication 10 sous forme d'une diode, d'une photodiode, d'un capteur, d'un transistor organique à effet de champ, d'un transistor pour des affichages flexibles, d'un RFID (radio-frequency identification device - dispositif d'identification par radiofréquence), ou d'une cellule solaire (à hétérojonction).

12. Utilisation d'un composé selon l'une quelconque des revendications 1 à 8 en tant qu'un semi-conducteur.

13. Procédé pour la préparation d'un dispositif semi-conducteur organique, la couche semi-conductrice organique étant formée par l'application d'une solution et/ou d'une dispersion d'un composé selon l'une quelconque des revendications 1 à 8 dans un solvant organique, sur un substrat approprié et l'élimination du solvant ; ou
la couche semi-conductrice organique étant formée par dépôt par évaporation sous vide d'un composé selon l'une quelconque des revendications 1 à 8.

14. Procédé pour la préparation d'un composé de formule 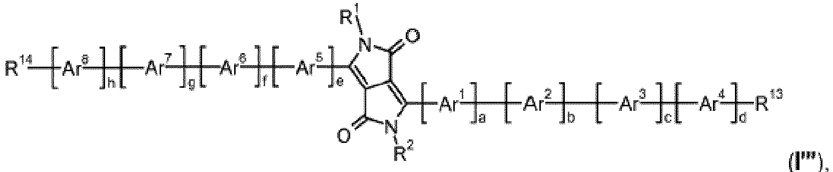 lequel procédé comprenant la mise en réaction d'un composé de formule 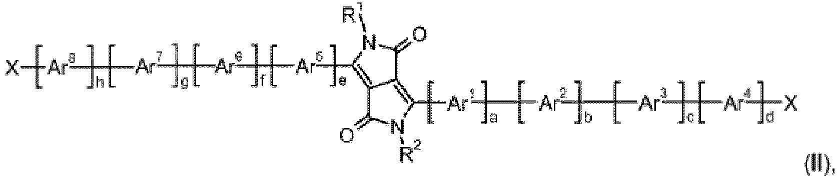 avec du cyanure de cuivre (I) ; ou procédé pour la préparation d'un composé de formule 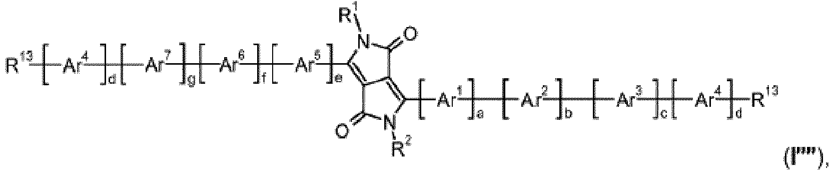 lequel procédé comprenant la mise en réaction d'un composé de formule 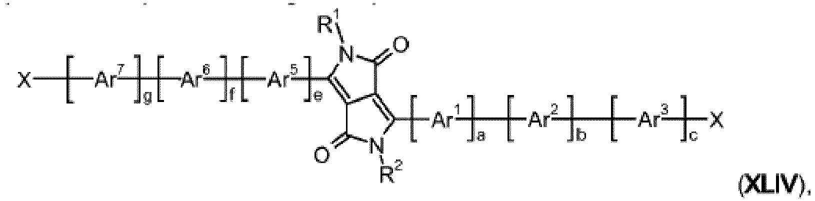 avec une quantité équimolaire d'un acide diboronique ou d'un diboronate correspondant à la formule X¹¹-A²-R¹³ dans un solvant et en présence d'un catalyseur, ou avec une quantité équimolaire d'un composé d'organoétain correspondant à la formule X^{11'}-A²-R¹³.
ou procédé pour la préparation d'un composé de formule 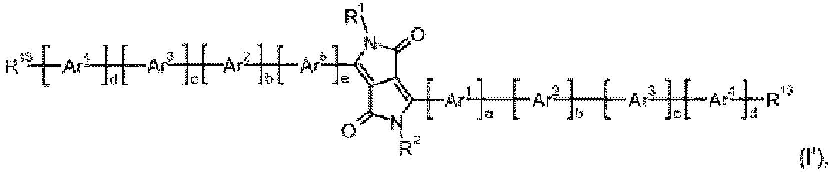 lequel procédé comprenant la mise en réaction d'un composé de formule 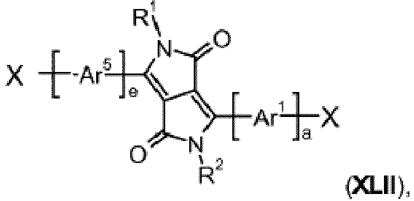 avec une quantité équimolaire d'un acide diboronique ou d'un diboronate correspondant à la formule X¹¹-A-R¹³ dans un solvant et en présence d'un catalyseur, ou avec une quantité équimolaire d'un composé d'organoétain correspondant à la formule X^{11'}-A-R¹³; ou procédé pour la préparation d'un composé de formule 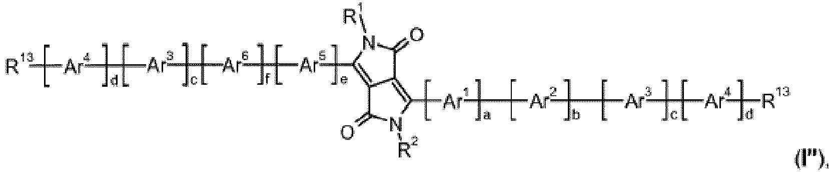 lequel procédé comprenant la mise en réaction d'un composé de formule 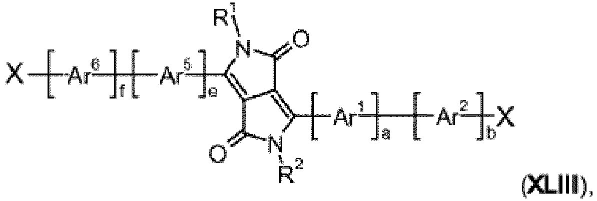 avec une quantité équimolaire d'un acide diboronique ou d'un diboronate correspondant à la formule X¹¹-A¹-R¹³ dans un solvant en présence d'un catalyseur, ou avec une quantité équimolaire d'un composé d'organoétain correspondant à la formule X^{11'}-A¹-R¹³ ; X¹¹ étant indépendamment en chaque occurrence -B(OH)₂, -B(OY¹)₂, 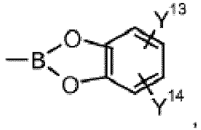 ou 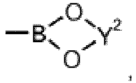 Y¹ étant indépendamment en chaque occurrence un groupe C₁₋₁₈-alkyle et Y² étant indépendamment en chaque occurrence un groupe C₂₋₁₀-alkylène, tel que -CY³Y⁴-CY⁵Y⁶-, ou -CY⁷Y⁸-CY⁹Y¹⁰-CY¹¹Y¹²-, dans lequel Y³, Y⁴, Y⁵, Y⁶, Y⁷, Y⁸, Y⁹, Y¹⁰, Y¹¹ et Y¹² est indépendamment les uns des autres hydrogène, ou un groupe C₁₋₁₈-alkyle, particulièrement -C(CH₃)₂C(CH₃)₂-,-C(CH₃)₂CH₂C(CH₃)₂-, ou -CH₂C(CH₃)₂CH₂-, et Y¹³ et Y¹⁴ étant indépendamment l'un de l'autre hydrogène, ou un groupe C₁₋₁₈-alkyle, dans un solvant et en présence d'un catalyseur ;
X^{11'} étant indépendamment en chaque occurrence-SnR²⁰⁷R²⁰⁸R²⁰⁹, R²⁰⁷, R²⁰⁸ et R²⁰⁹ étant identiques ou différents et étant H ou C₁₋₆-alkyle, ou deux des groupes R²⁰⁷, R²⁰⁸ et R²⁰⁹ formant un cycle et ces groupes étant éventuellement ramifiés,
R¹³ et R¹⁴ étant CN,
A étant un groupe de formule 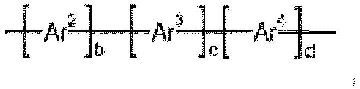 A¹ étant un groupe de formule 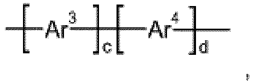 A² étant un groupe de formule 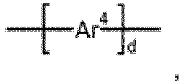 X étant Cl, Br, ou I ;
a étant 1, et e étant 1,
b, c, d, f, g et h représentant indépendamment les uns des autres 0 ou 1,
R¹, R², Ar¹, Ar², Ar³, Ar⁴, Ar⁵, Ar⁶, Ar⁷ et Ar⁸ étant tels que définis selon la revendication 1.
